# EUROPEAN PATENT SPECIFICATION

(11) **EP 3 087 198 B1**
(45) Date of publication and mention of the grant of the patent: **22.01.2020**
(21) Application number: 14820806.9
(22) Date of filing: 15.12.2014
(51) Int. Cl.: C12Q 1/6869, C12Q 1/689

(54) **METAGENOMIC ANALYSIS OF SAMPLES**
METAGENOMISCHE ANALYSE VON PROBEN
ANALYSE D'ÉCHANTILLONS MÉTAGÉNOMIQUES

(30) Priority: 24.12.2013 EP 13199610; 13.01.2014 EP 14150903
(43) Date of publication of application: 02.11.2016
(73) Proprietor: Université de Liège, 4031 Angleur (BE); Quality Partner S.A., 4040 Herstal (BE)
(72) Inventor: DAUBE, Georges, 4000 Liege (BE); TAMINIAU, Bernard, 4000 Liege (BE); NEZER, Carine, 4040 Herstal (BE); DELHALLE, Laurent, 4040 Herstal (BE)
(74) Representative: Barker Brettell LLP
(86) International application number: PCT/EP2014/077672
(87) International publication number: WO 2015/097006

(56) References cited:
- EUN-JIN PARK ET AL: "Bacterial community analysis during fermentation of ten representative kinds of kimchi with barcoded pyrosequencing", FOOD MICROBIOLOGY, ACADEMIC PRESS LTD, LONDON, GB, vol. 30, no. 1, 7 October 2011 (2011-10-07), pages 197-204, XP028445534, ISSN: 0740-0020, DOI: 10.1016/J.FM.2011.10.011 [retrieved on 2011-10-19]
- I. M. CARROLL ET AL: "Alterations in composition and diversity of the intestinal microbiota in patients with diarrhea-predominant irritable bowel syndrome", NEUROGASTROENTEROLOGY & MOTILITY, vol. 24, no. 6, 20 February 2012 (2012-02-20), pages 521-e248, XP055119317, ISSN: 1350-1925, DOI: 10.1111/j.1365-2982.2012.01891.x
- NOCKER ANDREAS ET AL: "Discrimination between live and dead cells in bacterial communities from environmental water samples analyzed by 454 pyrosequencing", INTERNATIONAL MICROBIOLOGY, SPRINGER VERLAG IBERICA, BARCELONA, ES, vol. 13, no. 2, 1 January 2010 (2010-01-01), pages 59-65, XP002671329, ISSN: 1139-6709, DOI: 10.2436/20.1501.01.111
- DEREK S LUNDBERG ET AL: "Practical innovations for high-throughput amplicon sequencing", NATURE METHODS, vol. 10, no. 10, 1 September 2013 (2013-09-01), pages 999-1002, XP055120581, ISSN: 1548-7091, DOI: 10.1038/nmeth.2634 & Derek S Lundberg ET AL: "Supplementary Information for: Practical innovations for high-throughput amplicon sequencing", Nature Methods, 1 September 2013 (2013-09-01), XP055143051, DOI: 10.1038/nmeth.2634 Retrieved from the Internet: URL:http://www.nature.com/nmeth/journal/v1 0/n10/extref/nmeth.2634-S1.pdf [retrieved on 2014-09-26]
- WINTZINGERODE VON F ET AL: "Peptide nucleic acid-mediated PCR clamping as a useful supplement in the determination of microbial diversity", APPLIED AND ENVIRONMENTAL MICROBIOLOGY, AMERICAN SOCIETY FOR MICROBIOLOGY, US, vol. 66, no. 2, 1 February 2000 (2000-02-01), pages 549-557, XP002310906, ISSN: 0099-2240, DOI: 10.1128/AEM.66.2.549-557.2000
- MASAO SAKAI ET AL: "Application of peptide nucleic acid (PNA)-PCR clamping technique to investigate the community structures of rhizobacteria associated with plant roots", JOURNAL OF MICROBIOLOGICAL METHODS, vol. 92, no. 3, 1 March 2013 (2013-03-01), pages 281-288, XP055141983, ISSN: 0167-7012, DOI: 10.1016/j.mimet.2012.09.036

### SEQUENCE LISTING

<110> Université de Liege and Quality Partner s.a.
<120> Metagenomic analysis of samples
<130> Daub2013-54
<150> EP13199610.0
   <151> 2013-12-24
<150> EP14150903.4
   <151> 2014-01-13
<160> 107
<170> PatentIn version 3.5
<210> 1
   <211> 21
   <212> DNA
   <213> Artificial Sequence
<220>
   <223> Primer
<400> 1
   gagagtttga tymtggctca g 21
<210> 2
   <211> 17
   <212> DNA
   <213> Artificial Sequence
<220>
   <223> Primer
<400> 2
   accgcggctg ctggcac 17
<210> 3
   <211> 82
   <212> DNA
   <213> Artificial Sequence
<220>
   <223> Consensus Sequence
<400> 3
   gagagttcga tcctggctca ggatgaacgc tggcggcatg cttaacacat gcaagtcgga 60
   cgggaagtgg tgtttccagt gg 82
<210> 4
   <211> 82
   <212> DNA
   <213> Artificial Sequence
<220>
   <223> Consensus Sequence
<400> 4
   gagagttcga tcctggctca ggatgaacgc tggcggcatg cttaacacat gcaagtcgga 60
   cgggaagtgg tgtttccagt gg 82
<210> 5
   <211> 82
   <212> DNA
   <213> Artificial Sequence
<220>
   <223> Consensus Sequence
<400> 5
   gagagtttga tcatggctca ggatgaacgc tggcggcatg cttaacacat gcaagtcgga 60
   cgggaagtgg tgtttccagt gg 82
<210> 6
   <211> 82
   <212> DNA
   <213> Artificial Sequence
<220>
   <223> Consensus Sequence
<400> 6
   gagagtttga tcatggctca ggatgaacgc tggcggcatg cttaacacat gcaagtcgga 60
   cgggaagtgg tgtttccagt gg 82
<210> 7
   <211> 82
   <212> DNA
   <213> Artificial Sequence
<220>
   <223> Consensus Sequence
<400> 7
   gagagtttga tcatggctca ggatgaacgc tggcggcatg cttaacacat gcaagtcgga 60
   cgggaagtgg tgtttccagt gg 82
<210> 8
   <211> 82
   <212> DNA
   <213> Artificial Sequence
<220>
   <223> Consensus Sequence
<400> 8
   gagagtttga tcatggctca ggatgaacgc tggcggcatg cttaacacat gcaagtcgga 60
   cgggaagtgg tgtttccagt gg 82
<210> 9
   <211> 82
   <212> DNA
   <213> Artificial Sequence
<220>
   <223> Consensus Sequence
<400> 9
   gagagtttga tcatggctca ggatgaacgc tggcggcatg cttaacacat gcaagtcgaa 60
   cgggaagtgg tgtttccagt gg 82
<210> 10
   <211> 82
   <212> DNA
   <213> Artificial Sequence
<220>
   <223> Consensus Sequence
<400> 10
   gagagtttga tcatggctca ggatgaacgc tggcggcatg cttaacacat gcaagtcgaa 60
   cgggaagtgg tgtttccagt gg 82
<210> 11
   <211> 94
   <212> DNA
   <213> Pseudomonas
<400> 11
   gagagtttga tcatggctca gattgaacgc tggcggcagg cctaacacat gtcaagtcga 60
   gcggatgaga agagcttgct cttcgattca gcgg 94
<210> 12
   <211> 101
   <212> DNA
   <213> Xanthomonas
<400> 12
   gagagtttga tcatggctca gagtgaacgc tggcggcagg cctaacacat gcaagtcgaa        60
   cggcagcggg taggaagctt gcttcctatg ccggcgagtg g                           101
<210> 13
   <211> 99
   <212> DNA
   <213> Escherichia coli
<400> 13
   gagagtttga tcatggctca gattgaacgc tggcggcagg cctaacacat gcaagtcgaa        60
   cggtaacagg aagcagcttg ctgtttcgct gacgagtgg                               99
<210> 14
   <211> 112
   <212> DNA
   <213> Lactococcus
<400> 14
   gagagtttga tcctggctca ggatgaacgc tggcggcgtg cctaatacat gcaagtcgaa        60
   cgaaacttct ttatcaccga gtgcttgcac tcaccgataa agagttgagt gg               112
<210> 15
   <211> 97
   <212> DNA
   <213> Brochothrix
<400> 15
   gagagtttga tcctggctca ggacgaacgc tggcggcgtg cctaatacat gcaagtcgaa        60
   cgaacggata aagagcttgc tcttttgaag ttagtgg                                 97
<210> 16
   <211> 91
   <212> DNA
   <213> Arthrobacter
<400> 16
   gagagtttga tcatggctca ggatgaacgc tggcggcgtg cttaacacat gcaagtcgaa        60
   cgatgaacct cacttgtggg gggattagtg g                                       91
<210> 17
   <211> 17
   <212> DNA
   <213> Artificial Sequence
<220>
   <223> Primer
<400> 17
   gaagtggtgt ttccagt 17
<210> 18
   <211> 65
   <212> DNA
   <213> Artificial Sequence
<220>
   <223> Consensus Sequence
<400> 18
   ggagaagaag caatgacggt atctggggaa taagcatcgg ctaactctgt gccagcagcc 60
   gcggt 65
<210> 19
   <211> 65
   <212> DNA
   <213> Artificial Sequence
<220>
   <223> Consensus Sequence
<400> 19
   ggagaagaag caatgacggt atctggggaa taagcatcgg ctaactctgt gccagcagcc 60
   gcggt 65
<210> 20
   <211> 65
   <212> DNA
   <213> Artificial Sequence
<220>
   <223> Consensus Sequence
<400> 20
   ggagaagaag caatgacggt atctggggaa taagcatcgg ctaactctgt gccagcagcc 60
   gcggt 65
<210> 21
   <211> 65
   <212> DNA
   <213> Artificial Sequence
<220>
   <223> Consensus Sequence
<400> 21
   ggagaagaag caatgacggt atctggggaa taagcatcgg ctaactctgt gccagcagcc 60
   gcggt 65
<210> 22
   <211> 65
   <212> DNA
   <213> Artificial Sequence
<220>
   <223> Consensus Sequence
<400> 22
   ggagaagaag caatgacggt atctggggaa taagcatcgg ctaactctgt gccagcagcc 60
   gcggt 65
<210> 23
   <211> 65
   <212> DNA
   <213> Artificial Sequence
<220>
   <223> Consensus Sequence
<400> 23
   agagaagaag caatgacggt atctggggaa taagcatcgg ctaactctgt gccagcagcc 60
   gcggt 65
<210> 24
   <211> 65
   <212> DNA
   <213> Artificial Sequence
<220>
   <223> Consensus Sequence
<400> 24
   ggagaagaaa caatgacggt atctgaggaa taagcatcgg ctaactctgt gccagcagcc 60
   gcggt 65
<210> 25
   <211> 65
   <212> DNA
   <213> Artificial Sequence
<220>
   <223> Consensus Sequence
<400> 25
   ggagaagaaa caatgacggt atctgaggaa taagcatcgg ctaactccgt gccagcagcc 60
   gcggt 65
<210> 26
   <211> 88
   <212> DNA
   <213> Pseudomonas
<400> 26
   tgggaggaag ggcagtaagc gaataccttg ctgttttgac gttaccgaca gaataagcac 60
   cggctaactc tgtgccagca gccgcggt 88
<210> 27
   <211> 88
   <212> DNA
   <213> Xanthomonas
<400> 27
   ggggaagaaa tcctgctggc taatatccgg cggggatgac ggtacccaaa gaataagcac 60
   cggctaactt cgtgccagca gccgcggt 88
<210> 28
   <211> 88
   <212> DNA
   <213> Escherichia coli
<400> 28
   ggggaggaag ggagtaaagt taataccttt gctcattgac gttacccgca gaagaagcac 60
   cggctaactc cgtgccagca gccgcggt 88
<210> 29
   <211> 88
   <212> DNA
   <213> Lactococcus
<400> 29
   agagaagaaa gtgcgtgaga gtaactgttc acgtttcgac ggtatctaac cagaaagcca 60
   cggctaacta cgtgccagca gccgcggt 88
<210> 30
   <211> 88
   <212> DNA
   <213> Brochothrix
<220>
   <221> misc_feature
   <222> (34)..(34)
   <223> n is a, c, g, or t
<400> 30
   agagaagaac atgggtgaga gtaactgttc accncttgac ggtatctaac cagaaagcca 60
   cggctaacta cgtgccagca gccgcggt 88
<210> 31
   <211> 79
   <212> DNA
   <213> Arthrobacter
<400> 31
   agggtagaag cctgtcttag gggtcggtga cggtacctgc agaagaagcg ccggctaact 60
   acgtgccagc agccgcggt 79
<210> 32
   <211> 17
   <212> DNA
   <213> Artificial Sequence
<220>
   <223> Primer
<400> 32
   arcaatgacg gtatctg 17
<210> 33
   <211> 45
   <212> DNA
   <213> Oryza sativa
<400> 33
   ctgtttgatg agcctgcgta gtattaggta gttggtcggg taaag 45
<210> 34
   <211> 45
   <212> DNA
   <213> Oryza rufipogon
<400> 34
   ctgtttgatg agcctgcgta gtattaggta gttggtcggg taaag 45
<210> 35
   <211> 45
   <212> DNA
   <213> Bambusa oldhamii
<400> 35
   ctgtttgatg agcctgcgta gtattaggta gttggtcagg taaag 45
<210> 36
   <211> 45
   <212> DNA
   <213> Triticum aestivum
<400> 36
   ctgtttgatg agcctgcgta gtattaggta gttggtcagg taaag 45
<210> 37
   <211> 45
   <212> DNA
   <213> S. cereale
<400> 37
   ctgtttgatg agcctgcgta gtattaggta gttggtcagg taaag 45
<210> 38
   <211> 45
   <212> DNA
   <213> Sorghum bicolor
<400> 38
   ctgtttgatg agcctgcgta gtattaggta gttggtcagg taaag 45
<210> 39
   <211> 46
   <212> DNA
   <213> Artificial Sequence
<220>
   <223> Consensus Sequence
<400> 39
   gcccgaggag gggctcgcgt ctgattagct agttggtgag gcaata 46
<210> 40
   <211> 46
   <212> DNA
   <213> Artificial Sequence
<220>
   <223> Consensus Sequence
<400> 40
   gcccgaggag gggctcgcgt ctgattagct agttggtgag gcaata 46
<210> 41
   <211> 46
   <212> DNA
   <213> Artificial Sequence
<220>
   <223> Consensus Sequence
<400> 41
   gcccaaggag gggctcgcgt ctgattagct agttggtgag gcaata 46
<210> 42
   <211> 46
   <212> DNA
   <213> Artificial Sequence
<220>
   <223> Consensus Sequence
<400> 42
   gcccaaggag gggctcgcgt ctgattagct agttggtgag gcaata 46
<210> 43
   <211> 47
   <212> DNA
   <213> Escherichia coli
<400> 43
   tgccatcgga tgtgcccaga tgggattagc ttgttggtgg ggtaacg 47
<210> 44
   <211> 50
   <212> DNA
   <213> Lactococcus lactis
<400> 44
   tatcactttg ggatggacct gcggcgcatt agcttgttgg tagggtaacg 50
<210> 45
   <211> 50
   <212> DNA
   <213> weissella
<400> 45
   tatcactaag agatggtccc gcggtgcatt agctagttgg taaggtaatg 50
<210> 46
   <211> 50
   <212> DNA
   <213> Brochothrix thermosphacta
<400> 46
   tgtcactgtg agatggaccc gcgctggatt agctagttgg taaggtaatg 50
<210> 47
   <211> 50
   <212> DNA
   <213> staphylococcus sp
<400> 47
   tatcacttat agatggaccc gcgccgtatt agctagttgg taaggtaacg 50
<210> 48
   <211> 18
   <212> DNA
   <213> Artificial Sequence
<220>
   <223> Primer
<400> 48
   tgtttgatga gcctgcgt 18
<210> 49
   <211> 49
   <212> DNA
   <213> Lactobacillus delbrueckii
<400> 49
   ggcctaccaa ggcaatgatg cgtagccgag ttgagagact gatcggcca 49
<210> 50
   <211> 49
   <212> DNA
   <213> Lactobacillus delbrueckii
<400> 50
   ggcctaccaa ggcaatgatg cgtagccgag ttgagagact gatcggcca 49
<210> 51
   <211> 49
   <212> DNA
   <213> Lactobacillus amylovorus
<400> 51
   ggcttaccaa ggcgacgatg catagccgag ttgagagact gatcggcca 49
<210> 52
   <211> 49
   <212> DNA
   <213> Lactobacillus acidophilus
<400> 52
   ggcctaccaa ggcaatgatg catagccgag ttgagagact gatcggcca 49
<210> 53
   <211> 49
   <212> DNA
   <213> Lactobacillus amyloticus
<400> 53
   ggcttaccaa ggcaacgatg catagccgag ttgagagact gatcggcca 49
<210> 54
   <211> 49
   <212> DNA
   <213> Lactobacillus acetotolerans
<400> 54
   ggcttaccaa ggcaacgatg catagccgag ttgagagact gatcggcca 49
<210> 55
   <211> 49
   <212> DNA
   <213> Lactococcus piscium
<400> 55
   ggactaccaa ggcgatgata catagccgac ctgagagggt gatcggcca 49
<210> 56
   <211> 49
   <212> DNA
   <213> Lactococcus plantarum
<400> 56
   ggactaccaa ggcgatgata catagccgac ctgagagggt gatcggcca 49
<210> 57
   <211> 49
   <212> DNA
   <213> Lactococcus chungangensis
<400> 57
   ggactaccaa ggcgatgata catagccgac ctgagagggt gatcggcca 49
<210> 58
   <211> 49
   <212> DNA
   <213> Lactococcus raffinolactis
<400> 58
   ggactaccaa ggcgatgata catagccgac ctgagagggt gatcggcca 49
<210> 59
   <211> 49
   <212> DNA
   <213> Lactococcus-lactis-subsp-Hordn
<400> 59
   ggctcaccaa ggcgatgata catagccgac ctgagagggt gatcggcca 49
<210> 60
   <211> 49
   <212> DNA
   <213> Lactococcus-lactis-subsp-Lacti
<400> 60
   ggctcaccaa ggcgatgata catagccgac ctgagagggt gatcggcca 49
<210> 61
   <211> 49
   <212> DNA
   <213> Lactococcus-lactis-subsp-Cremo
<400> 61
   ggctcaccaa ggcgatgata catagccgac ctgagagggt gatcggcca 49
<210> 62
   <211> 49
   <212> DNA
   <213> Streptococcus thermophilus
<400> 62
   ggctcaccaa ggcgacgata catagccgac ctgagagggt gatcggcca 49
<210> 63
   <211> 49
   <212> DNA
   <213> Streptococcus thermophilus
<400> 63
   ggctcaccaa ggcgatgata catagccgac ttgagagggt gatcggcca 49
<210> 64
   <211> 49
   <212> DNA
   <213> Streptococcus thermophilus
<400> 64
   ggctcaccta ggcgacgata catagccgac ctgagagggt gatcggcca 49
<210> 65
   <211> 49
   <212> DNA
   <213> Streptococcus parauberis
<400> 65
   ggctcaccaa ggccacgata catagccgac ctgagagggt gatcggcca 49
<210> 66
   <211> 49
   <212> DNA
   <213> Streptococcus iniae
<400> 66
   ggctcaccaa ggcgacgata catagccgac ctgagagggt gatcggcca 49
<210> 67
   <211> 49
   <212> DNA
   <213> Streptococcus dysgalactiae
<400> 67
   ggctcaccaa ggcgacgata catagccgac ctgagagggt gatcggcca 49
<210> 68
   <211> 49
   <212> DNA
   <213> Streptococcus agalactiae
<400> 68
   ggctcaccaa ggcgacgata catagccgac ctgagagggt gatcggcca 49
<210> 69
   <211> 49
   <212> DNA
   <213> Streptococcus equi
<400> 69
   ggcctaccaa ggcgacgata catagccgac ctgagagggt gaacggcca 49
<210> 70
   <211> 49
   <212> DNA
   <213> Streptococcus oligofermentans
<400> 70
   ggctcaccta ggcgacgata catagccgac ctgagagggt gatcggcca 49
<210> 71
   <211> 49
   <212> DNA
   <213> Enterococcus faecalis
<220>
   <221> misc_feature
   <222> (11)..(11)
   <223> n is a, c, g, or t
<220>
   <221> misc_feature
   <222> (13)..(13)
   <223> n is a, c, g, or t
<400> 71
   ggctcaccaa ngncacgatg catagccgac ctgagagggt gatcggcca 49
<210> 72
   <211> 49
   <212> DNA
   <213> Lactobacillus algidus
<400> 72
   ggcccaccaa ggcaatgata cgtagccgac ctgagagggt gatcggcca 49
<210> 73
   <211> 49
   <212> DNA
   <213> Lactobacillus frumenti
<400> 73
   ggcttaccaa ggcgatgatg catagccgag ttgagagact gatcggcca 49
<210> 74
   <211> 49
   <212> DNA
   <213> Lactobacillus panis
<400> 74
   ggcttaccaa ggcaatgatg catagccgag ttgagagact gatcggcca 49
<210> 75
   <211> 49
   <212> DNA
   <213> Lactobacillus fermentum
<400> 75
   ggcctaccaa ggcgatgatg catagccgag ttgagagact gatcggcca 49
<210> 76
   <211> 49
   <212> DNA
   <213> Lactobacillus oligofermentans
<400> 76
   ggctcaccaa gacaatgata cgtagccgaa ctgagaggtt gatcggcca 49
<210> 77
   <211> 49
   <212> DNA
   <213> Lactobacillus plantarum
<400> 77
   ggctcaccat ggcaatgata cgtagccgac ctgagagggt aatcggcca 49
<210> 78
   <211> 49
   <212> DNA
   <213> Brevibacterium linens
<400> 78
   ggcctaccaa ggcgacgacg ggtagccggc ctgagagggc gaccggcca 49
<210> 79
   <211> 49
   <212> DNA
   <213> Psychroflexus sp
<400> 79
   ggcttaccat ggcaacgata ggtaggggtc ctgagaggga gatccccca 49
<210> 80
   <211> 18
   <212> DNA
   <213> Artificial Sequence
<220>
   <223> Primer
<400> 80
   gatgatacat agccgacc 18
<210> 81
   <211> 1501
   <212> DNA
   <213> Escherichia coli
<400> 81
   gagagtttga tcatggctca gattgaacgc tggcggcagg cctaacacat gcaagtcgaa      60
   cggtaacagg aagcagcttg cttctttgct gacgagtggc ggacgggtga gtaatgtctg     120
   ggaaactgcc tgatggaggg ggataactac tggaaacggt agctaatacc gcataacgtc     180
   gcaagaccaa agagggggac cttcgggcct cttgccatcg gatgtgccca gatgggatta     240
   gctagtaggt ggggtaacgg ctcacctagg cgacgatccc tagctggtct gagaggatga     300
   ccagccacac tggaactgag acacggtcca gactcctacg ggaggcagca gtggggaata     360
   ttgcacaatg ggcgcaagcc tgatgcagcc atgccgcgtg tatgaagaag gccttcgggt     420
   tgtaaagtac tttcagcggg gaggaaggga gtaaagttaa tacctttgct cattgacgtt     480
   acccgcagaa gaagcaccgg ctaactccgt gccagcagcc gcggtaatac ggagggtgca     540
   agcgttaatc ggaattactg ggcgtaaagc gcacgcagcg gtttgttaag tcagatgtga     600
   aatccccggg ctcaacctgg gaactgcatc tgatactggc aagcttgagt ctcgtagagg     660
   ggggtagaat tccaggtgta gcggtgaaat gcgtagagat ctggaggaat accggtggcg     720
   aaggcggccc cctggacgaa gactgacgct caggtgcgaa agcgtgggga gcaaacagga     780
   ttagataccc tggtagtcca cgccgtaaac gatgtcgact tggaggttgt gcccttgagg     840
   cgtggcttcc ggagctaacg cgttaagtcg accgcctggg gagtacggcc gcaaggttaa     900
   aactcaaatg aattgacggg ggcccgcaca agcggtggag catgtggttt aattcgatgc     960
   aacgcgaaga accttacctg gtcttgacat ccacggaagt tttcagagat gagaatgtgc    1020
   cttcgggaac cgtgagacag gtgctgcatg gctgtcgtca gctcgtgttg tgaaatgttg    1080
   ggttaagtcc cgcaacgagc gcaaccctta tcctttgttg ccagcggtcc ggccgggaac    1140
   tcaaaggaga ctgccagtga taaactggag gaaggtgggg atgacgtcaa gtcatcatgg    1200
   cccttacgac cagggctaca cacgtgctac aatggcgcat acaaagagaa gcgacctcgc    1260
   gagagcaagc ggacctcata aagtgcgtcg tagtccggat tggagtctgc aactcgactc    1320
   catgaagtcg gaatcgctag taatcgtgga tcagaatgcc acggtgaata cgttcccggg    1380
   ccttgtacac accgcccgtc acaccatggg agtgggttgc aaaagaagta ggtagcttaa    1440
   ccttcgggag ggcgcttacc actttgtgat tcatgactgg ggtgaagtcg taacaaggta    1500
   a                                                                    1501
<210> 82
   <211> 51
   <212> DNA
   <213> Artificial Sequence
<220>
   <223> Primer
<400> 82
   cctatcccct gtgtgccttg gcagtctcag gagagtttga tymtggctca g 51
<210> 83
   <211> 58
   <212> DNA
   <213> Artificial Sequence
<220>
   <221> misc_feature
   <222> (31)..(40)
   <223> n is a, c, g, or t
<400> 83
   ccatctcatc cctgcgtgtc tccgactcag nnnnnnnnnn taccgcggct gctggcac 58
<210> 84
   <211> 17
   <212> DNA
   <213> Artificial Sequence
<220>
   <223> Primer
<400> 84
   agactgggat aactccg 17
<210> 85
   <211> 18
   <212> DNA
   <213> Artificial Sequence
<220>
   <223> Primer
<400> 85
   aacctgcctt gtagcggg 18
<210> 86
   <211> 18
   <212> DNA
   <213> Artificial Sequence
<220>
   <223> Primer
<400> 86
   taacaacatg aatcgcat 18
<210> 87
   <211> 20
   <212> DNA
   <213> Artificial Sequence
<220>
   <223> Lactobacillus-Tuf-F2 primer
<400> 87
   gcycacgtwg aataygaaac 20
<210> 88
   <211> 23
   <212> DNA
   <213> Lactobacillus
<400> 88
   cgdacttcca tttcaacyaa gtc 23
<210> 89
   <211> 23
   <212> DNA
   <213> Lactobacillus
<400> 89
   tgtggcatwg grccatcagt tgc 23
<210> 90
   <211> 20
   <212> DNA
   <213> Lactococcus
<400> 90
   gccgaaatyg atggygaaat 20
<210> 91
   <211> 23
   <212> DNA
   <213> Lactococcus
<400> 91
   caactttttc acgcaattgg ttg 23
<210> 92
   <211> 24
   <212> DNA
   <213> Lactococcus
<400> 92
   tgatgtcwca agcyatgcgt aaac 24
<210> 93
   <211> 24
   <212> DNA
   <213> Leuconostoc
<400> 93
   ttcttgttcc atgaaatcca tttg 24
<210> 94
   <211> 22
   <212> DNA
   <213> Leuconostoc
<400> 94
   gaatacccac tagawcgtac ac 22
<210> 95
   <211> 29
   <212> DNA
   <213> Leuconostoc
<400> 95
   tgtgtttcac caattttgtg aattttacc 29
<210> 96
   <211> 17
   <212> DNA
   <213> Photobacterium
<400> 96
   aatgccaggt tgtgctg 17
<210> 97
   <211> 26
   <212> DNA
   <213> Photobacterium
<400> 97
   gacttgttca gagtwagaat wacttc 26
<210> 98
   <211> 25
   <212> DNA
   <213> Photobacterium
<400> 98
   atcgayggtg tgttacatga gtaca 25
<210> 99
   <211> 17
   <212> DNA
   <213> Pseudomonas
<400> 99
   ggcgtkatcg aytcsgt 17
<210> 100
   <211> 19
   <212> DNA
   <213> Pseudomonas
<400> 100
   cagmggrcgc tggttgatg 19
<210> 101
   <211> 18
   <212> DNA
   <213> Pseudomonas
<400> 101
   tcgygttgcy gaygacga 18
<210> 102
   <211> 20
   <212> DNA
   <213> Carnobacterium
<400> 102
   attggygtat taccagtcga 20
<210> 103
   <211> 19
   <212> DNA
   <213> Carnobacterium
<400> 103
   aaccatctgc ccatacatc 19
<210> 104
   <211> 23
   <212> DNA
   <213> Carnobacterium
<400> 104
   cgatttacac cccagttagt cgt 23
<210> 105
   <211> 20
   <212> DNA
   <213> Brochotrix
<400> 105
   cagtaccttc tggtaacgtg 20
<210> 106
   <211> 24
   <212> DNA
   <213> Brochotrix
<400> 106
   acttctttca ctgcagaagt atac 24
<210> 107
   <211> 30
   <212> DNA
   <213> Brochotrix
<400> 107
   tattatctaa agaagagggt ggacgtcata 30

## Description

The invention relates to an analytical method that has been developed to identify and quantify the total microorganism content of fermented food samples.

Microbial contents of food, faeces or environmental samples are frequently either identified or quantified through culture dependant methods. Among new generation molecular technologies the metagenomic analysis has emerged as a powerful tool. Metagenomics applies a suite of genomic technologies and bioinformatics tools to directly access the genetic content of entire communities of organisms. This technology is commonly used to describe microbial community profiles of various ecosystems like seas, soils, rumen, and faeces but also to study complex microbiota of foodstuff.

Several problems remain with metagenomic analysis of foodstuffs and other like samples. Firstly, it is necessary to provide a global metagenomic approach which is not dependant on the microbe's ability to grow on determined culture media at any define environmental conditions (e.g. pH, T°, Aw). Such an approach should provide for the description as well as quantification of the observed microorganisms, which is of critical needs in some environments, for example when food spoilage issues are to be explained.

Secondly, prior art methods of metagenomic analysis may be biased by the presence in the samples being tested of non-viable microorganisms. In many cases, particularly in the metagenomic analysis of food samples, it is important to be able to characterise and quantify only the live microorganisms in a sample. Prior art metagenomic techniques do not allow for such an analysis and results, when applied to food sample analysis may therefore be misleading.

Thirdly, prior art methods of metagenomic analysis may be biased by the presence of contaminating DNA present in the sample, which is amplified together with the target sequence of interest, but which does not provide useful information on the microbiota of the sample. For example chloroplast DNA or mitochondrial DNA may co-amplify with the target, where the target is the well known 16S rRNA gene.

Fourthly, prior art methods of metagenomic analysis may be biased by the presence of a large amount of DNA from bacteria or other microorganisms deliberately added to foodstuff as an ingredient or by the presence of a natural dominant species. Such DNA can mask the DNA of interest in a metagenomic analysis.

Park EJ et al. (Food microbiology, Academic Press Ltd, London, GB, Vol. 30, no.1, pages 197-204, 2011) describes bacterial community analysis during fermentation of ten representative kinds of kimchi with barcoded pyrosequencing.

Carrol IM et al. (Neurogastroenterology & Motility, Vol. 24, no. 6, pages 521-e248, 2012) describes alterations in composition and diversity of the intestinal microbiota in patients with diarrhea-predominant irritable bowel syndrome.

Nocker A et al. (International Microbiology, Springer Verlag Iberica, Barcelona, ES, Vol. 13, no. 2, pages 59-65, 2010) describes discrimination between live and dead cells in bacterial communities from environmental water samples analyzed by 454 pyrosequencing.

Lundberg DS et al. (Nature Methods, page 999, 2013) describes improvements for sequencing 16S ribosomal RNA (rRNA) amplicons, a cornerstone technique in metagenomics.

There is therefore a need for improved metagenomic analysis methods and tools. The invention overcomes these and other problems.

### Summary of the Invention

According to a first aspect of the invention there is provided a method of metagenomic analysis of fermented food in accordance with claim 1 herein.

Described is an integrated metagenomic approach developed to (I) identify and (II) quantify the micro-organism content of any samples, (III) distinguish if the observed DNA sequences derived from dead or alive germs, (IV) block the well known co-amplification of mitochondrial and plastid DNA with universal bacterial 16S primers, that might represent more than 90 % of the sequences in some food preparations, and (V) block the amplification of over-represented germs avoiding rare but important germs to be observed.

Our metagenomic analysis is a PCR-based approach targeting the highly conserved rDNA locus encoding for the 16S rDNA gene. This gene is commonly used for bacterial taxonomic classification as it contains a succession of nine hyper variable regions with well-conserved sequences in between. The conserved sequences are used to design universal bacterial primers flanking a polymorphic region chosen based on its high information content for a precise species identification of microorganism content.

Described is a method of metagenomic analysis of a food or stool sample to detect and quantitate microorganisms present in said sample, the analysis comprising amplification of the V2-V3 region of bacterial 16S rRNA in said sample. In some embodiments the V1-V3 region of bacterial 16S rRNA is amplified. The inventors have identified that the V2-V3 region provides excellent hypervariability between common food spoilage flora, and therefore allows for bacteria likely to be present in food samples to be distinguished and quantitated.

In some embodiments, the use of V2-V3 of 16S rRNA, according to the invention, in a metagenomic analysis, allows for the identification and distinguishing of sequences from bacteria of the families: *Acetobacteraceae, Aeromonadaceae, Bacteroidaceae, Burkholderiaceae, Carnobacteriaceae, Clostridiaceae, Comamonadaceae, Enterobacteriaceae, Enterococcaceae, Flavobacteriaceae, Lachnospiraceae, Lactobaillaceae, Leuconostocaceae, Listeriaceae, Moraxellaceae, Neisseriaceae, Pseudoalteromonadaceae, Pseudomonadaceae, Shewanellaceae, Streptococcaceae, Vibrionaceae* and *Xanthomonadaecea.*

In more detail, the invention allows for the detection and distinguishing of bacteria of the genera: *Acetobacteraceae Acetobacter, Acetobacteraceae Gluconacetobacter, Aeromonadaceae Aeromonas, Bacteroidaceae Bacteroides, Burkholderiaceae Burkholderia, Carnobacteriaceae Carnobacterium, Clostridiaceae Clostridium, Comamonadaceae Comamonas, Comamonadaceae Delftia, Enterobacteriaceae Escherichia, Enterobacteriaceae Pantoea, Enterobacteriaceae Serratia, Enterococcaceae Enterococcus, Chryseobacterium, Flavobacteriaceae Flavobacterium, Flavobacteriaceae Myroides, Lachnospiraceae Butyrivibrio, Lactobaillaceae Lactobacillus, Lactobacillaceae Pediococcus, Leuconostocaceae Leuconostoc, Leuconostocaceae Weissella, Listeriaceae Listeria, Moraxellaceae Acinetobacter, Moraxellaceae Psychrobacter, Neisseriaceae Alysiella, Pseudoalteromonadaceae Pesudoalteromonas, Pseudomonadaceae Pseudomonas, Shewanellaceae Shewanella, Streptococcaceae Lactococcus, Streptococcaceae Streptococcus, Vibrionaceae Aliivibrio, Vibrionaceae Photobacterium, Vibrionaceae Vibrio* and *Xanthomonadaecea Xanthomonas.*

Metagenomic analysis of the DNA present in a sample may provide a relative quantitative analysis of different microorganisms present. However in preferred embodiments the amount of some, or each of the microorganisms detected is normalised and expressed as a percentage of the colony forming units in the sample. This may be important in some embodiments since the absolute relative amounts of microorganisms in a sample may not be indicative of the spoilage potential of the sample or the shelf life limit. Therefore one may accurately qualify the quality of a food sample.

Normalisation of the quantity of microorganisms in a sample may be carried out by determining the percentage of microorganisms represented by at least one operational taxonomic unit (OTU) in the sample. This may be combined with determining the colony forming units count (CFU) of the sample and normalising the percentage of organisms represented by said OTU to a proportion of the total viable CFU count of the sample.

Further quantitation of DNA in the sample may be provided, in some embodiments, by conducting taxa specific quantitative PCR (qPCR) on samples following metagenomic analysis. In such embodiments, the taxa chosen for the qPCR analysis are those taxa of interest identified by the metagenomic analysis. Any form of qPCR may be employed using any suitable gene, and any detection and quantification process. For example the 16S rRNA gene may be amplified in qPCR tests. Different qPCR tests that might be employed include those involving an intercalating dye, and those involving a labelled reporter primer or probe. For example qPCR may be conducted using a hydrolysis probe (e.g. a Taqman probe), a molecular beacon probe, a pair of dual hybridisation probes, an amplifluour primer system, a scorpion primer system, a light-upon-extension system or a QZyme primer system.

In some embodiments the V2-V3 region of 16S rRNA is amplified in the metagenomic analysis as part of a larger fragment of the 16S rRNA gene. For example, in some embodiments, the V1-V3 region is amplified.

In some embodiments one or both primers having sequences comprising the sequences of SEQ ID NOs 1 and/or 2 may be used to amplify the VI-V3 region. Preferably, a pair of primers comprising the sequences of SEQ ID NO. 1 and SEQ ID NO. 2 respectively are used to amplify the VI-V3 region. In some embodiments the primers consist of the sequences of SEQ ID NOs 1 and 2 respectively.

Such primers may be modified by the addition of adaptors, key sequences, tags or universal sequences. In some embodiments, at least one of the primers has the sequence of SEQ ID NO. 82. In some embodiments, at least one of the primers as the sequence of SEQ ID NO. 83. In some embodiments a pair of primers having the sequences of SEQ ID NO.s 82 and 83 respectively is used.

In order to improve the analytical power of metagenomic analysis of a sample, described are methods in which the amplification of plastid or mitochondrial DNA, and/or DNA from selected microorganisms is reduced. Described is the use of Peptide Nucleic Acid (PNA) clamping in a method of metagenomic analysis of a sample, to reduce amplification of at least one of: i) chloroplast DNA present in the sample; ii) mitochondrial DNA present in the sample; or iii) DNA from one or more selected microorganisms that may be present in the sample.

The PNA clamping may comprise conducting metagenomic analysis in the presence of a PNA oligonucleotide comprises a sequence derived from a chloroplast 16S rRNA gene, preferably a sequence common to a plurality of chloroplast 16S rRNA genes.

The PNA oligonucleotide may comprise a sequence common to at least two of, and preferably all of, the chloroplast 16S rRNA sequences of SEQ ID NOs 3 to 10, or common to at least two of, and preferably all of, the chloroplast 16S rRNA sequences of SEQ ID NOs 18 to 25.

The PNA oligonucleotide may have a sequence not found in the bacterial 16S rRNA sequences of at least one of, and preferably all of, SEQ ID NOs 11 to 16, or has a sequence not found in the bacterial 16S rRNA sequences of at least one of, and preferably all of, SEQ ID NOs 26 to 31.

The PNA oligonucleotide may comprise the sequence of SEQ ID NO. 17. Preferably the PNA oligonucleotide consists of the sequence of SEQ ID NO. 17.

The PNA oligonucleotide may comprise the sequence of SEQ ID NO. 32. Preferably, the PNA oligonucleotide consists of the sequence of SEQ ID NO. 32.

The metagenomic analysis may be conducted in the presence of a pair of PNA oligonucleotides comprising of or consisting of the sequences of SEQ ID NO.s 17 and 32 respectively.

The PNA clamping may comprise conducting metagenomic analysis in the presence of a PNA oligonucleotide comprising a sequence derived from a mitochondrial 18S rRNA gene, preferably a sequence common to a plurality of mitochondrial 18S rRNA genes.

The PNA oligonucleotide may comprise a sequence common to at least two of, and preferably all of, the mitochondrial 18S rRNA sequences of SEQ ID NOs 33 to 38.

The PNA oligonucleotide may have a sequence not found in the chloroplast 16S rRNA sequences, or the bacterial 16S rRNA sequences of at least one of, and preferably all of, SEQ ID NOs 39 to 47.

The PNA oligonucleotide may comprise the sequence of SEQ ID NO. 48, optionally wherein said PNA oligonucleotide consists of the sequence of SEQ ID NO. 48.

In some embodiments, the PNA clamping comprises conducting metagenomic analysis in the presence of a PNA oligonucleotide comprising a sequence derived from a bacterial 16S rRNA gene, preferably a sequence common to a plurality of bacterial 16S rRNA genes.

The PNA oligonucleotide may comprise a sequence common to at least two of, and preferably all of, the bacterial 16S rRNA sequences of SEQ ID NOs 49 to 61.

The PNA oligonucleotide may have a sequence not found in the bacterial 16S rRNA sequences of at least one of, and preferably all of, SEQ ID NOs 62 to 79.

In some embodiments, the PNA oligonucleotide comprises the sequence of SEQ ID NO. 80, optionally wherein said PNA oligonucleotide consists of the sequence of SEQ ID NO. 80.

The metagenomic analysis may be conducted in the presence of any two, three or four of the PNA oligonucleotides discussed above, that is any two, three or four PNA oligonucleotides comprising or consisting of the sequences of SEQ ID NO.s 17, 32, 48 or 80 respectively.

The invention is further directed towards the ability to distinguish the amplification of DNA from live and dead microorganisms in a metagenomic analysis of a sample. The invention achieves this by the use of the intercalating dye propidium monoazide which penetrates dead cells in the sample prior to DNA extraction, intercalates with the DNA of those dead cells and prevents subsequent amplification. The invention therefore provides in a further aspect, a method of metagenomic analysis of a sample, wherein propidium monoazide (PMA) is added to the sample prior to DNA extraction in order to reduce the subsequent amplification of DNA from non-viable microorganisms present in the sample.

In each and every one of the uses of PNA clamping described herein or the use of PMA as described herein, the metagenomic analysis may be carried out in accordance with any other embodiment or aspect of the invention described herein.

According to another aspect of the present invention there is provided a kit for metagenomic analysis of a sample according to claim 11 herein.

Described is a kit for metagenomic analysis of a sample comprising one or both primers comprising the sequence of SEQ ID NO. 1 and/or SEQ ID NO. 2, preferably wherein the primers consist of the sequences of SEQ ID NO. 1 and/or SEQ ID NO. 2.

In some embodiments, the primers of the kit may be modified by the addition of an adaptor, key sequence, tag or universal sequence. Preferably, the primers have the sequences of SEQ ID NO.s 82 and 83 respectively.

In some embodiments, a kit of the invention further comprises a PNA oligonucleotide comprising the sequence of SEQ ID NOs. 80

In some embodiments the kit of the invention may further comprise PMA.

In some embodiments the kit of the invention may further comprise a reagent for metagenomic analysis of a sample, e.g. a food or stool sample.

The invention will now be described in more detail with reference to the following illustrative Examples and the figures, in which:
Figure 1 shows the nine hypervariable regions of the 16S rRNA gene.
Figure 2 shows taxonomic information content along the whole 16S gene.
Figure 3 shows the sequence of *Escherichia coli* 16S ribosomal RNA gene (Genebank Accession Number AY804014.1, SEQ ID NO. 81) with annotation of the polymorphism frequencies across the bacterial kingdom.
Figure 4 shows primer statistics of both primers and amplicon product size, and the sequences of the forward and reverse primers (SEQ ID NO. 1 and SEQ ID NO. 2 respectively).

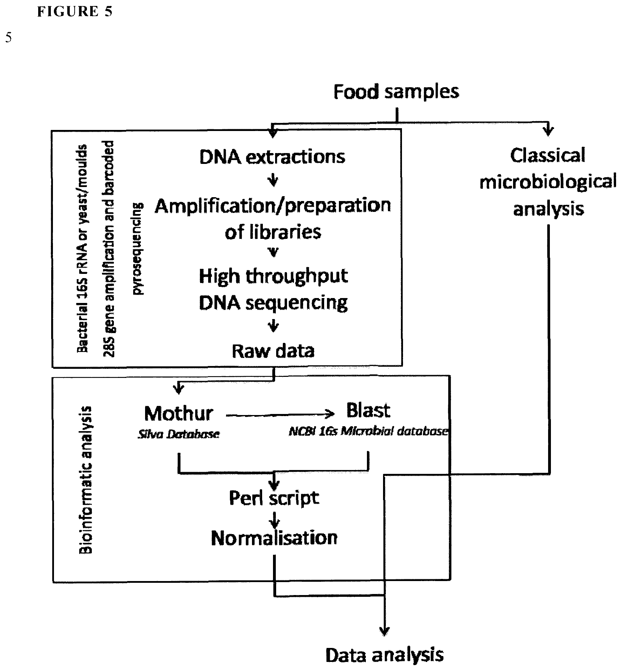

Figure 5 shows a flow diagram of the steps involved in the metagenomic analysis
Figure 6 shows an alignment of chloroplast 16S RNA sequences with various bacterial 16S rRNA sequences and the sequence of a first PNA clamp. Sequences entitled Chloroplast 1 to Chloroplast 8 are SEQ ID NO.s 3 to 10 respectively. SEQ ID NO.s of the other sequences are: *Pseudomonas* SEQ ID NO. 11; *Xanthomonas* SEQ ID NO. 12; *E. coli* SEQ ID NO. 13; *Lactococcus* SEQ ID NO. 14; *Brochothrix* SEQ ID NO. 15; *Artrhrobacter* SEQ ID NO 16. the PNA oligonucleotide QP-PNAcup is SEQ ID NO. 17.
Figure 7 shows a further alignment of chloroplast 16S RNA sequences with various bacterial 16S rRNA sequences and the sequence of a second PNA clamp. Sequences entitled Chloroplast 1 to Chloroplast 8 are SEQ ID NO.s 18 to 25 respectively. SEQ ID NO.s of the other sequences are: *Pseudomonas* SEQ ID NO. 26; *Xanthomonas* SEQ ID NO. 27; *E. coli* SEQ ID NO. 28; *Lactococcus* SEQ ID NO. 29; *Brochothrix* SEQ ID NO. 30; *Artrhrobacter* SEQ ID NO 31. the PNA oligonucleotide QP-PNAcdn is SEQ ID NO. 32.

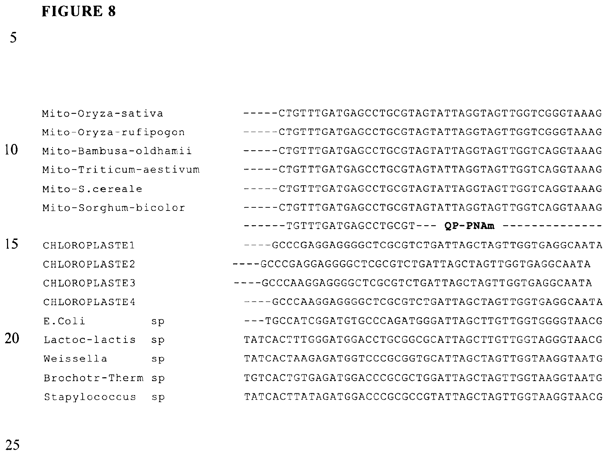

Figure 8 shows an alignment of mitochondrial 18S RNA sequences with various bacterial 16S rRNA sequences and the sequence of a third PNA clamp. SEQ ID NOs. of the sequences shows are: *Mito-Oryza-sativa* SEQ ID NO. 33; *Mito-Oryza-rufipogon* SEQ ID NO. 34; *Mito-Bambusa-oldhamii* SEQ ID NO. 35; *Mito-Triticum-aestivum* SEQ ID NO. 36; *Mito-S.cereale* SEQ ID NO.37; *Mito-Sorghum-bicolor* SEQ ID NO. 38; Chloroplast E1 to Chloroplast E4 are SEQ ID NOs. 39 to 42 respectively; *E. coli* SEQ ID NO. 43; *Lactococcus lactis* SEQ ID NO. 44; *Weissella* SEQ ID NO. 45; *Brochotrix thermosphacta* SEQ ID NO. 46; *Staphylococcus* SEQ ID NO.47. The PNA oligonucleotide QP-PNAm is SEQ ID NO. 48.

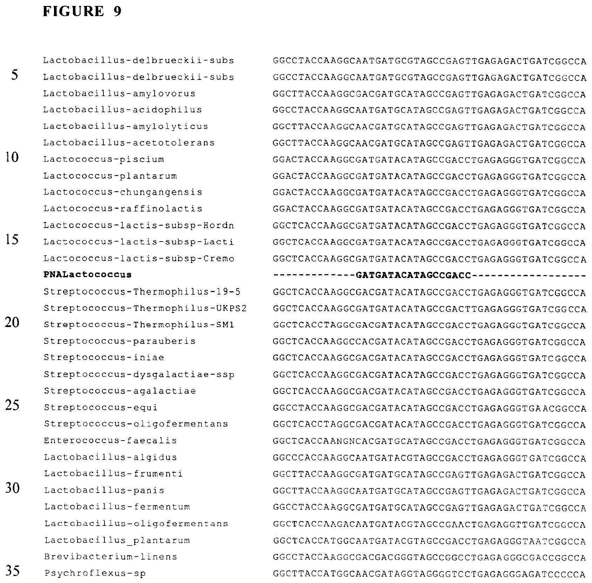

Figure 9 shows an alignment of 16S rRNA sequences from various bacteria of the lactococcus taxa, together with various contaminating bacteria, and a fourth PNA clamp. The SEQ ID NOs. of the sequences shown are: *Lactobacillus-delbrueckii-subs* SEQ ID NO.49; *Lactobacillus-delbrueckii-subs* SEQ ID NO. 50; *Lactobacillus-amylovorus* SEQ ID NO. 51; *Lactobacillus-acidophilus* SEQ ID NO. 52; *Lactobacillus-amylolyticus* SEQ ID NO. 53; *Lactobacillus-acetotolerans* SEQ ID NO. 54; *Lactococcus-piscium* SEQ ID NO. 55; *Lactococcus-plantarum* SEQ ID NO. 56; *Lactococcus-chungangensis* SEQ ID NO. 57; *Lactococcus-raffinolactis* SEQ ID NO. 58; *Lactococcus-lactis-subsp-Hordn* SEQ ID NO. 59; *Lactococcus-lactis-subsp-Lacti* SEQ ID NO. 60; *Lactococcus-lactis-subsp-Cremo* SEQ ID NO. 61; *Streptococcus-Thermophilus-19-5* SEQ ID NO. 62; *Streptococcus-Thermophilus-UKPS2* SEQ ID NO. 63; *Streptococcus-Thermophilus-SM1* SEQ ID NO. 64; *Streptococcus-parauberis* SEQ ID NO. 65; *Streptococcus-iniae* SEQ ID NO. 66; *Streptococcus-dysgalactiae-ssp* SEQ ID NO. 67; *Streptococcus-agalactiae* SEQ ID NO. 68; *Streptococcus-equi* SEQ ID NO. 69; *Streptococcus-oligofermentans* SEQ ID NO. 70; *Enterococcus-faecalis* SEQ ID NO. 71; *Lactobacillus-algidus* SEQ ID NO.72; *Lactobacillus-frumenti* SEQ ID NO. 73; *Lactobacillus-panis* SEQ ID NO.74; *Lactobacillus-fermentum* SEQ ID NO. 75; *Lactobacillus-oligofermentans* SEQ ID NO. *76; Lactobacillus_plantarum* SEQ ID NO. 77; *Brevibacterium-linens* SEQ ID NO. 78 *Psychroflexus-sp* SEQ ID NO. 79. The PNA oligonucleotide PNALactococcus is SEQ ID NO. 80.
Figure 10 shows metagenomic analysis of steak tartare samples.
Figure 11 shows relative proportions of the major bacterial taxa (over 1% of prevalence) in steak tartare samples.
Figure 12 shows a representation of the converted relative bacterial count of the OTUs in total count numbers of population. Only taxa whose population reached 10,000 cells per gram are visible. The bold bar indicates the threshold of 6 logs of CFUs above which the spoilage activity would be noticeable.
Figure 13 shows a comparison between bacterial content of 3 probiotics with and without PMA treatment.
Figure 14 shows an analysis of a pasta product with the standard metagenomic protocol, with the 2 PNA clamps specific to avoid plastid DNA, with the PNA directed against the mitochondrial DNA and finally with the 3 PNA clamps together.
Figure 15 shows bacterial profiles of 2 cheeses obtained with and without the use of a PNA *Lactococcus* clamping probe.

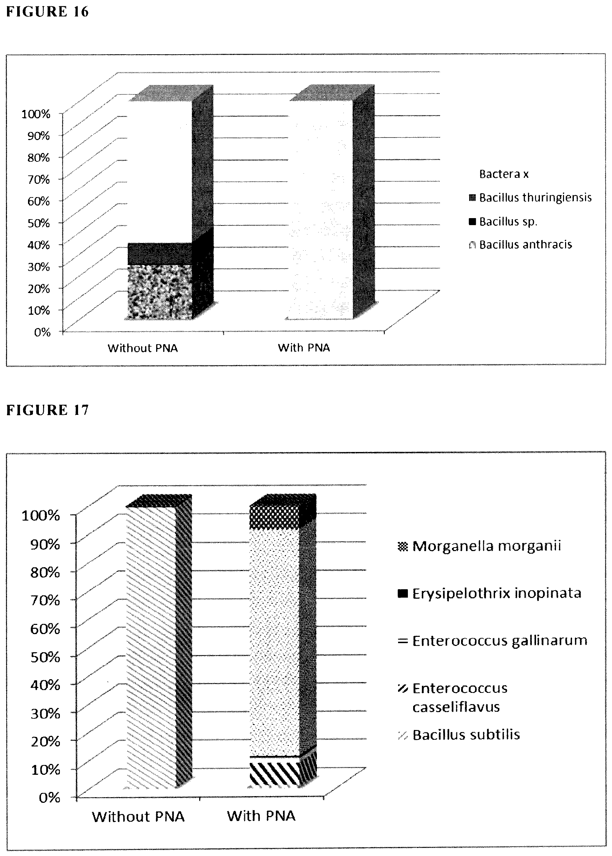

Figure 16 shows amplification of DNA from samples with and without PNA. In both the bacillus that are present in high concentration on the metagenomic profile are not amplified with the adjunction of the specific PNA design against a specific bacillus sequence.
Figure 17 shows amplification of DNA from samples with and without PNA. In both the bacillus that are present in high concentration on the metagenomic profile are not amplified with the adjunction of the specific PNA design against a specific bacillus sequence.

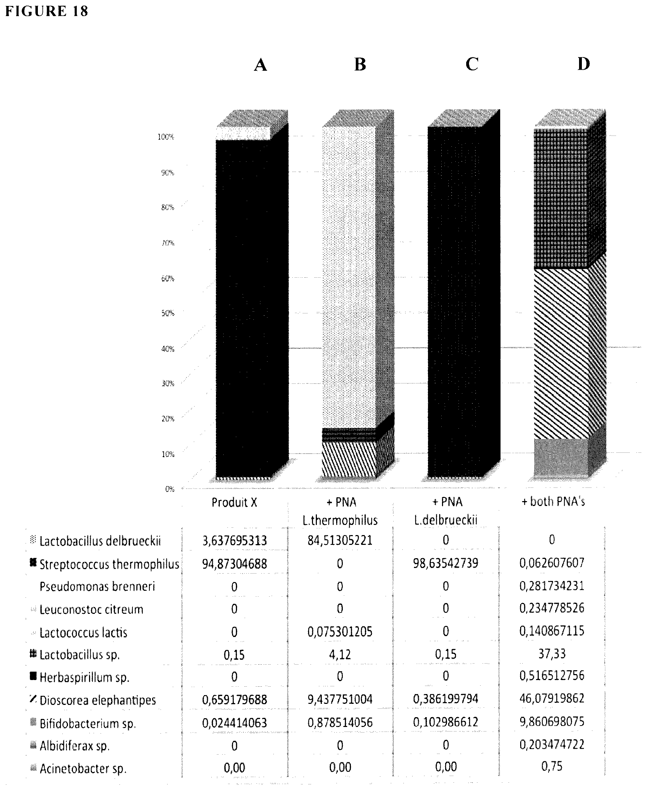

Figure 18 shows **Analysis A** shows the metagenomic result from a yoghurt product without any PNA use where more than 98 % of the sequences are coming from streptococcus thermophilus (94.9 %) and lactobacillus delbrueckii (3.6 %) leaving less place for the observation of real contamination floras.
   **Analysis B** shows the metagenomic result with the use of the specific PNA blocking the amplification of any DNA originating from streptococcus thermophilus. In this case, the second technologic flora is taking the lead with the presence of 84.5 % of the sequences originating from lactobacillus delbrueckii.
   **Analysis C** shows the metagenomic result with the use of the specific PNA blocking the amplification of any DNA originating from lactobacillus delbrueckii.. In this case, the second technologic flora is taking the lead with the presence of 98.6 % of the sequences originating from streptococcus thermophiles.
   **Analysis D** shows the metagenomic result with the use of the specific PNA blocking the amplification of any DNA originating from both technologic species (streptococcus thermophilus and lactobacillus delbrueckii). In this case only, the flora of interest is visible as a result of the metagenomic analysis of the food product.

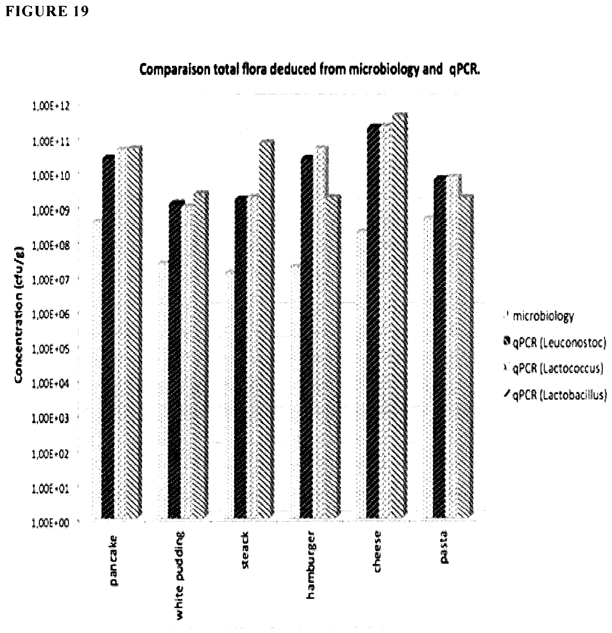

Figure 19 shows a comparaison of total flora estimated by classic microbiology and deduced by targeted qPCR for *Leuconostoc* sp, *Lactobacillus* sp and *Lactococcus* sp.

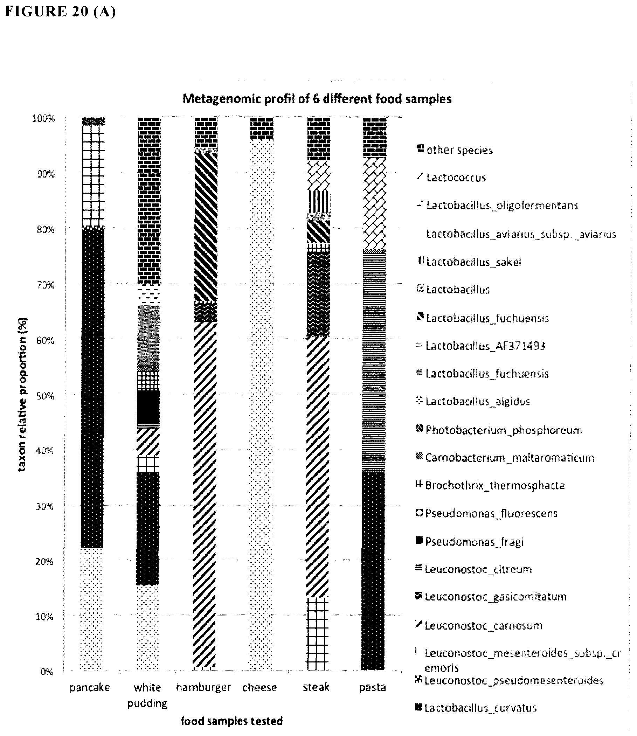

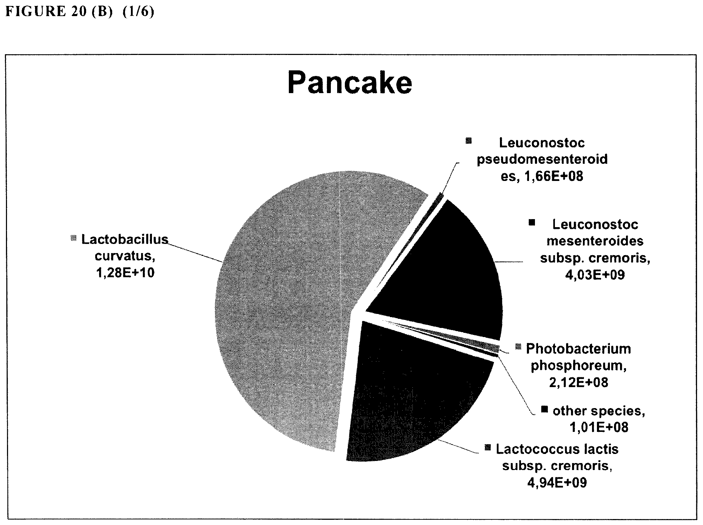

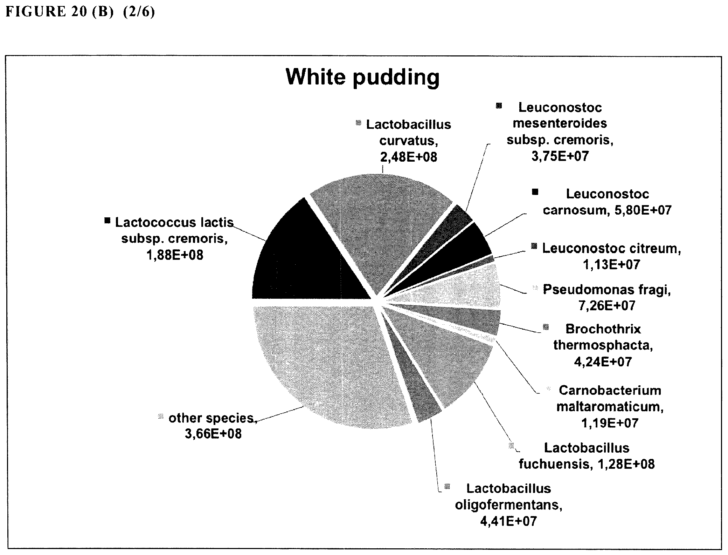

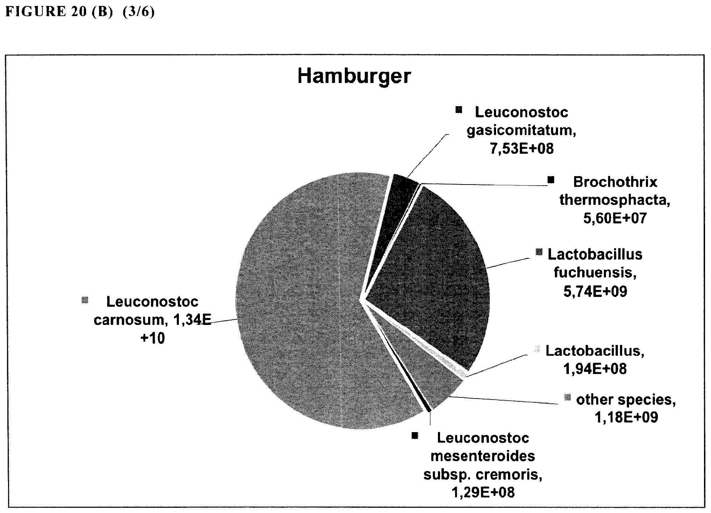

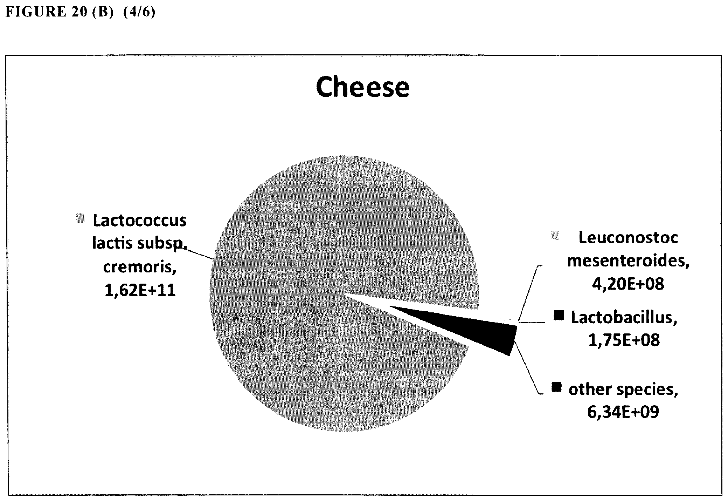

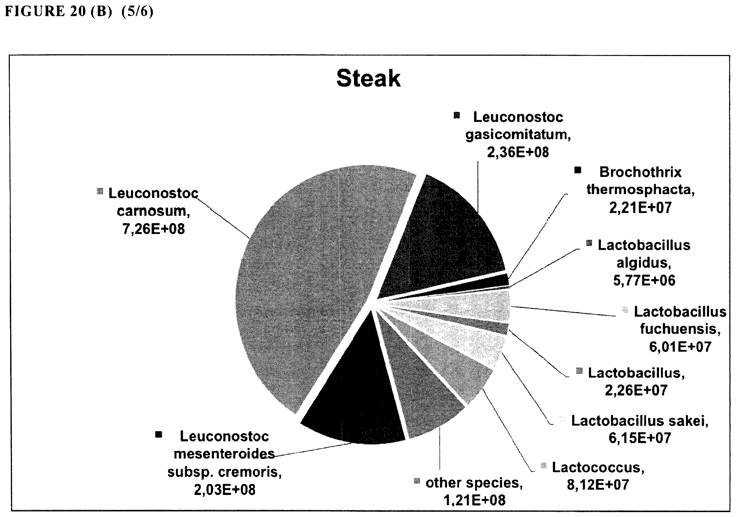

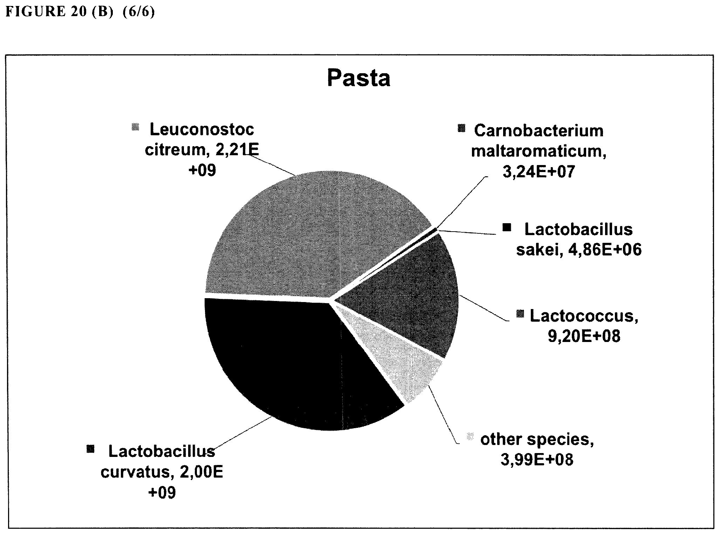

Figure 20 shows (A): Estimation (in percentage) of major taxon relative proportions by metagenomic analysis; (B): Estimation of each taxon concentration (in cfu/g) using the total flora estimation based on quantitative PCR.

### Examples

### Material and methods

### DNA isolation

Twenty-five gram sample of meat product are homogenized for 1 minute in 225 ml in a tempo bag (bioMérieux Basingstoke, England, ref 80015) with sterile physiologic water using a stomacher apparatus. 1.5 ml of the suspension are then used for total genomic DNA extraction using a homemade lysis step prior to the conventional use of the DNeasy Blood & Tissue Kit (QIAGEN, Crawley, UK). The DNA concentration is then evaluated by fluorometric dsDNA quantification using PicoGreen.

### Descriptive metagenomic analysis

### - Amplicon selection and primer designs

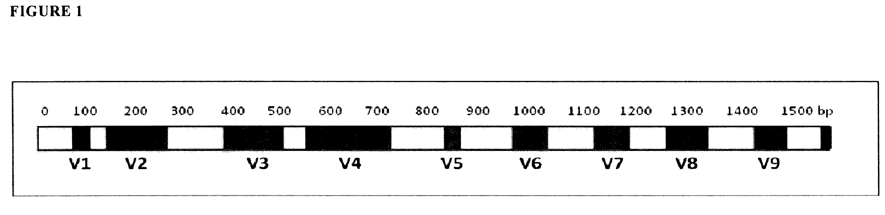

Culture-independent 16S rRNA gene sequencing has been widely applied to examine microbial diversity. Bacterial 16S ribosomal RNA (rRNA) genes contain nine "hypervariable regions" (VI-V9) (Figure 1), that demonstrate considerable sequence diversity among different bacteria. Unfortunately, 16S rRNA hypervariable regions exhibit different degrees of sequence diversity, and no single hypervariable region is able to distinguish among all bacteria. The full-length 16S rRNA genes (about 1500 bp) can be used for accurate taxonomic identification based on the underlying sequence diversity among different bacterial species. However, the current high-throughput DNA sequencing technologies can only produce 16S rRNA gene fragments, instead of full-length genes. The challenge is to obtain reliable representation of bacterial communities through taxonomic classification of short 16S rRNA gene sequences.

The inventors have discovered the most appropriate region for food metagenomic analysis. Information content of 16S rRNA, for bacterial assignation to the species level, were analyzed for different 16S regions across the most frequent food taxa.

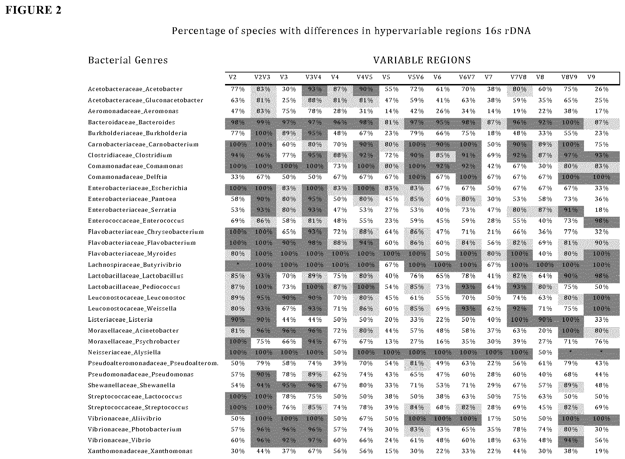

For this purpose, reference sequences from the most common food bacterial taxa were extracted from open databases (Silva) and in silico cut in various fragment containing the sequences of a single or two contagious hypervariable regions. After comparing those fragments to the databases, percentages of taxonomical identification to the species were computed for each region. As can be seen in figure 2, the V2-V3 region seems to be the more informative with respect to the common food spoilage flora.

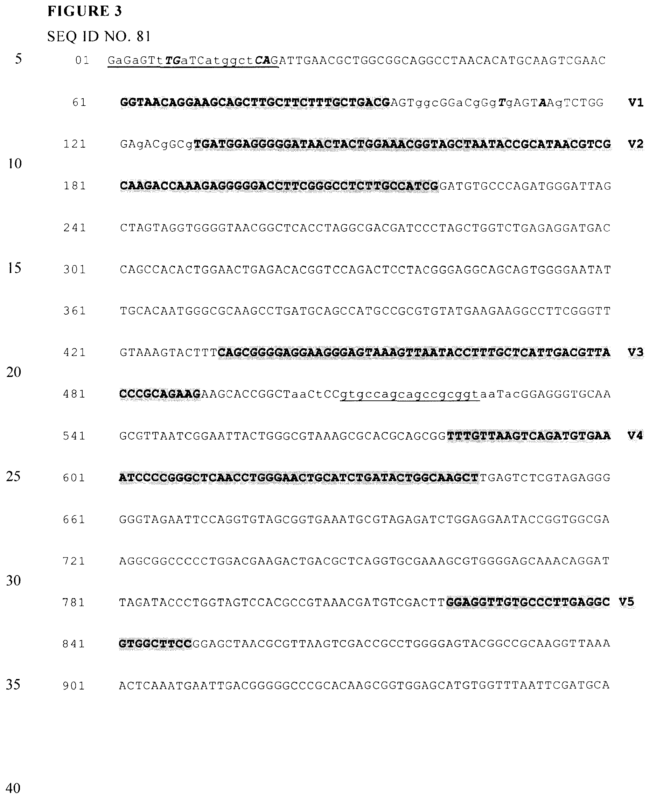

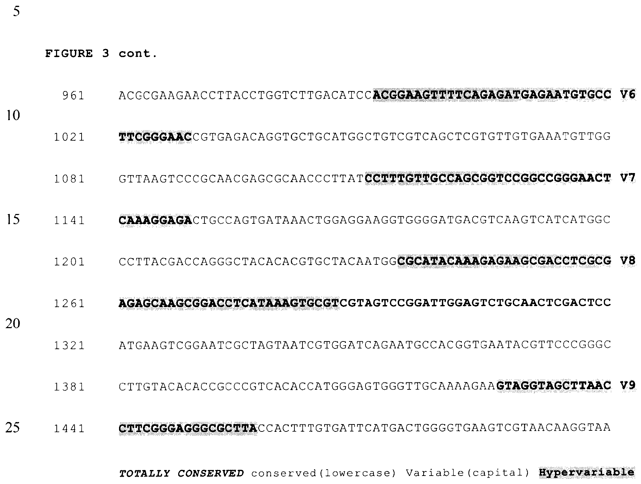

Given the small number of base pairs contained between V1 and V2 and their low level of conservation, the forward primer was designed upstream of V1 (Figure 3).

The presence of polymorphisms in the selected primers might cause a failure to detect some bacterial species and consequently lead to an incomplete picture of the microbiote composition characterization.

For this purpose, different "in silico" analysis have been driven (data not shown) and have led to the degeneration of 2 bases in the 16S forward primer (figure 3).

The final oligonucleotide design included 454 Life Sciences A or B sequencing titanium adaptors (Roche Diagnostics, Vilvoorde, Belgium) and key sequence fused to the 5' end of the specific target primers. The multiplex identifiers (MIDs) are only included along with the A adaptors.

As seen in Figure 3, two bases has been degenerated due to observed polymorphism across genera (results not shown).

### 16S gene library construction and pyrosequensing

The bacterial VI-V3 regions of the 16S rRNA gene were amplified by PCR using a homemade fusion primer set leading to an amplicon of 625 bp in length. For the GS Junior Titanium chemistry, the forward primer (5'-CCTATCCCCTGTGTGCCTTGGCAGTCTCAGGAGAGTTTGATYMTGGCTCAG-3') (SEQ ID NO. 82) contain the B adaptor, a key sequence (TCAG) and our forward conserved bacterial primer (Roche, Basel, Switzerland). The reverse primer (5'-CCATCTCATCCCTGCGTGTCTCCGACTCAGNNNNNNNNNNTACCGCGGCTGCTG GCAC-3') (SEQ ID NO 83) contain the A adaptor, a key sequence (TCAG), ten bases barcodes (Ns) to distinguish samples and our reverse conserved bacterial primer.

The amplification mix contained 5 U of FastStart high fidelity polymerase (Roche Diagnostics, Vilvoorde, Belgium), 1x enzyme reaction buffer, 200 µM dNTPs (Eurogentec, Liege, Belgium), 0.2 µM of each fusionprimer and genomic DNA in a volume of 25 µl.

The PCR conditions are as follows: 4 min at 94 °C, 25 cycles of denaturation (15 sec at 94 °C), annealing (45 s at 56 °C) and extension (60 s at 72 °C), followed by a final elongation (8 min at 72 °C). These amplifications were performed on an Ep Master system gradient apparatus (Eppendorf, Hamburg, Germany).

The PCR products of 625 nucleotides are checked by gel electrophoresis and purified using the AMPure kit (Agencourt Bioscience Corporation, Beverly, USA) to remove amplicons shorter than 100 bp. Equal amounts of each of the PCR products are pooled and subsequently amplified by emulsion PCR before sequencing. Pyrosequencing is then performed with the Roche 454 GS Junior Sequencer (Roche, Basel, Switzerland) using the massively parallel pyrosequencing protocol.

### Bioinformatic analysis

Image and data processing for amplicon sequencing are performed using the Genome Sequencer FLX System Software Package 2.3 (Roche, Basel, Switzerland). The Mothur program is used for the first part of the sequences analysis. Sequences with a length under 450 bases are discarded. The sequences with ambiguous bases or homopolymers longer than ten nucleotides and with an average quality score lower than 25 are removed along with tag and primer sequences. Sequences from multiplexed samples are assigned based on the presence of the unique barcodes assigned to each sample. The sequences that pass the quality check are aligned to the SILVA alignment database. Chimeric sequences are detected using the chimera-slayer command included in the Mothur package, and the potential chimeras are removed. The sequences are preclustered to reduce false operational taxonomic units produced by erroneous sequences. A distance matrix is prepared, (distance= 0.01) and the sequences are clustered to operational taxonomic units (OTUs) using the average neighbour algorithm. An 80% confidence threshold is required for genus level assignment with the SILVA Database. OTU representing less than 0.01% of the total number of reads are considered as artefacts and discarded.

In a second phase, the representative sequences of each OTU are compared to the NCBI 16S microbial database for example using the Basic Local Alignment Search Tool (BLASTN). The BLAST assignments for each OTU are assigned if the BLASTN default score is above 800. The genus assignations obtained by Mothur and by BLAST are compared and noted as unclassified in a case of mismatch. In cases of several hits with a score above 800, the first hit with the highest score is fostered to give the bacterial species, and 5 following hits with a decreasing score are given for complementary information on the BLAST results.

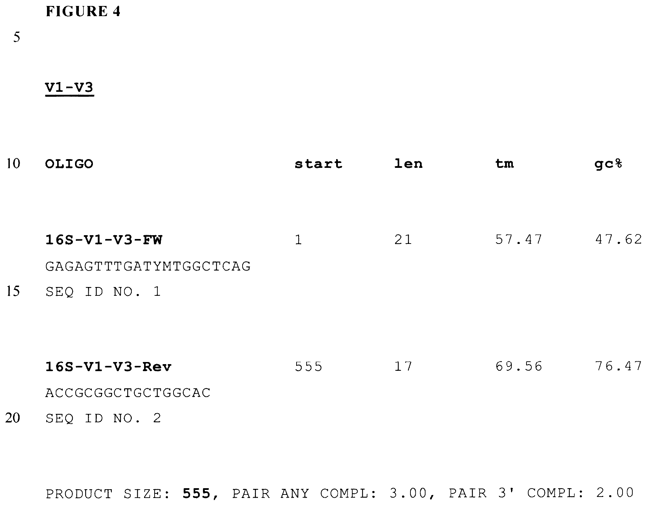

Finally, conversion and normalization of data are carried out. The number of sequences from each OTU is used to compute percentages. The percentage of each OTU is finally converted as a proportion of the total viable count obtained by classical microbiological analysis. Figure 4 gives the flow diagram of the metagenomic analysis of any sample.

The complete description of the bioinformatic homemade pipeline is described in annexe 1 which is herein incorporated in its entirety.

### Semi-quantitative metagenomic analysis

Conversion of sequence proportions into real colony forming units is of great importance especially for the food sector. For a microorganism to induce organoleptic disorders, its average concentration has to reach minimum 10^{5_6} CFU per gram of sample. Indeed, saying that a spoilage organism represents even 90 percent of the observed bacteria does not mean anything if just a low amount of total flora is present in the product. Normalization of the sequences numbers in a log CFU estimation is therefore important.

Microbiological analyses are performed using a classic microbiological approach for the determination of the total bacterial count and, in some extent, the enumeration of bacterial sub-populations.

Ten-fold dilution series of the homogenized samples are prepared in peptone water and plated onto the corresponding medium. Total bacterial count is measured by counting the colony forming units (CFUs) present on plate count agar (PCA) (Oxoid, Dardilly, France) after 48 h of incubation at 30 °C. The results are expressed in logarithms of CFU per gram of sample.

The percentage of each OTU might then be converted as a proportion of the total viable count obtained by classical microbiological analysis.

As metagenomic analysis is a non-cultured based method, DNA derived from dead bacteria is also sequenced. Sequences normalization on the total growing bacterial count is introducing an underestimation. To counteract this bias, qPCR targeting diverse taxa may be provided.

### Discrimination between dead or alive bacteria

Metagenomic analyses have revolutionized microbial detection. However the obstacle for DNA-based techniques is that DNA in the environment can be very stable and can persist for extended periods of time (days to weeks) after cell death. As DNA from dead or alive microorganisms is PCR amplified in the same way, this will lead to an overestimation of microbial targets. One of the prerequisites of making ecological conclusions derived from genetic fingerprints is that bacterial community profiles reflect the live portion of the sample of interest.

Sample treatment with Propidium monoazide (PMA), a DNA intercalating agent, prior to DNA extraction successfully avoids amplification of targets from dead microorganisms.

Propidium monoazide is a membrane-impermeant dye that selectively penetrates cells with compromised membranes, which can be considered dead. Once inside the cells, PMA intercalates into the DNA and can be covalently cross-linked to it after exposure to strong visible light. This subsequent complex strongly inhibits further PCR amplification. By using PCR after PMA treatment, the analysis of bacterial communities can theoretically be limited to cells with intact cell membranes.

Twenty-five gram of food sample are homogenized for 1 minute in 225 ml in a tempo bag (bioMérieux Basingstoke, England, ref 80015) with sterile physiologic water using a stomacher apparatus. 1.5 ml of the suspension are then centrifuge. After discarding the supernatant, the pellet is exposed for 10 minutes to PMA treatment (50 µM) in a dark room.

After a 5 minutes light exposure, the solution is centrifuge again and the pellet is directly mixed with the lysis solution prior to DNA extraction using DNeasy Blood & Tissue Kit (QIAGEN, Crawley, UK).

### Elimination of plastid and mitochondrial DNA co-amplification.

In case of prepared food samples containing spices, herbs, fungi, vegetables or fruits; whatever the extraction method used to isolate DNA from these their bacterial communities, the extracted microbial DNA is generally contaminated with DNA of plant origin, in particular chloroplasts and mitochondria. The same problem will occur for any 16S metagenomic analysis aiming to learn about plant-associates microbiotes.

Since the chloroplast 16S and mitochondrial 18S rRNA genes share high sequence similarity with bacterial 16S rRNA sequences contamination with plant DNA poses a serious challenge for the application of PCR-based methods to profile and quantify bacterial populations in plant environments.

Without specific treatment, it is not rare to sequence more than 95 percent of mitochondrial and chloroplastic DNA instead of the bacterial one that is actually targeted, which is quite irrelevant, and undesired considering the price of a metagenome analysis.

PNA (Peptide Nucleic Acid), an artificially created DNA analogue, was invented by Drs. Nielsen, Egholm, Berg, and Buchardt in 1991. The phosphate ribose ring of DNA was replaced with the polyamide backbone in PNA. Despite a radical structural change, PNA is capable of sequence-specific binding in a helix form to its complementary DNA or RNA sequence. Due to its superior binding affinity and chemical/biological stability, PNA has been widely applied in the field of biology (www.panagen.com).

PNA PCR clamping technique was developed very early to detect SNP in low abundance. For example, a few mutant cancerous cells may be among healthy cells in a tissue biopsy. PNA clamp complementary to wild type sequence hybridizes specifically with the wild type and blocks its PCR amplification while allowing amplification of mutant sequence of imperfect match. A single base mismatch is enough to discriminate amplification of mutant type from wild type.

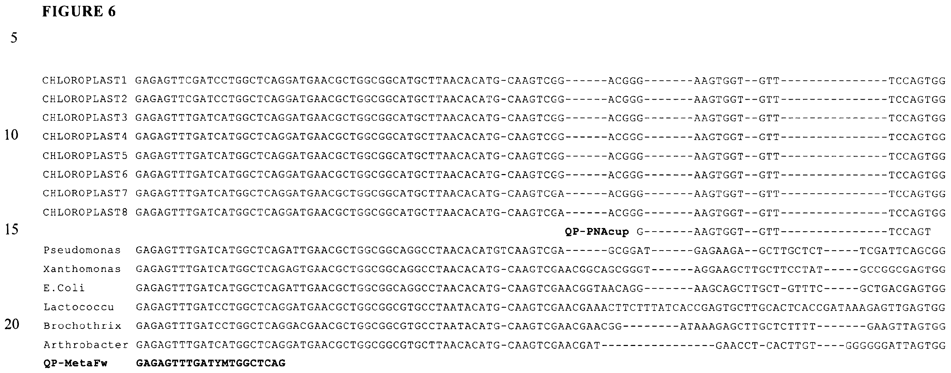

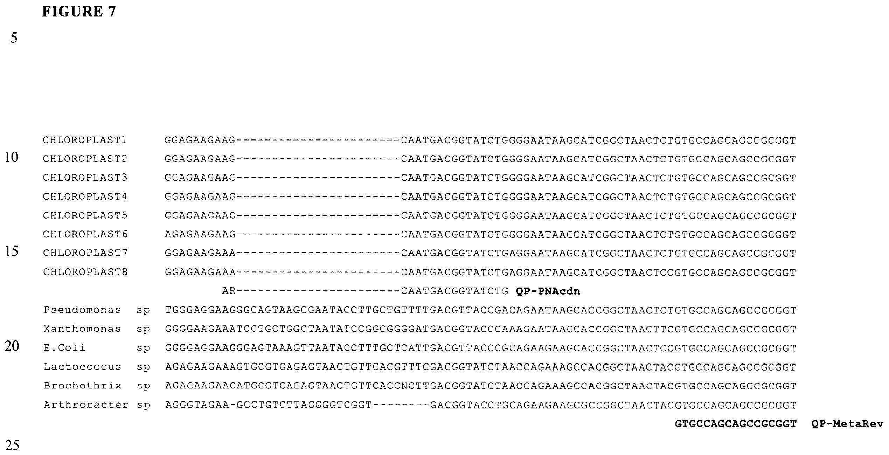

Two PNA clamps have been developed to block the co-amplification of the chloroplastic DNA (Figures 6 and 7).

A third PNA clamp has been developed to block the co-amplification of the mitochondrial DNA (Figure 8).

The use of those 3 PNA clamps in the PCR mix allow to mainly amplify the desired bacterial 16S DNA for the microbiome profiling assay.

### Elimination of specific undesired over-represented taxa.

In some cases, the bacterial diversity might be hidden by an over represented taxa.
One example is the presence of a huge amount of *Lactococcus* species in any cheese as these taxa are extensively used as starter in the production of buttermilk and cheese. Specific PNA clamping probes have been designed to avoid the amplification of any bacteria belonging to the *Lactococcus* taxa in order to give emphasis on the environmental contaminating flora or on any other species contributing to the specific texture, flavour or odour of the cheese.

On the same principal of the mitochondrial or plastid design, we founded a PNA sequence between our metagenomic V1-V3 primers. The PNA probe is characterized by a sequence conserved for example across the *Lactococcus* taxa but showing at least one point mutation compare to the other bacterial classes frequently observed in cheese products.

The alignment used for the *lactococcus* PNA probe is represented in Figure 9.

### Results

### Descriptive metagenomic analysis

The present invention might be used for a complete description of the total flora of any sample (food, beverage, stools, any biological sample, soil, water or any environment sample).

As an example of a descriptive metagenomic analysis, we analyse a set of steak tartare samples. Steak tartare is indeed a popular meat dish in Belgium and other European countries. This meat preparation, due to its raw nature, is highly sensitive to bacterial spoilage. A better understanding of the bacterial content of this product will thus be insightful to control the risk of spoilage. Metagenomics targeted on the 16S ribosomal DNA has appeared as a powerful tool to study bacterial composition of food samples. The aim of this study is to identify the bacterial populations of steak tartare from different origins along their shelf life and determine the main spoilage bacterial species.

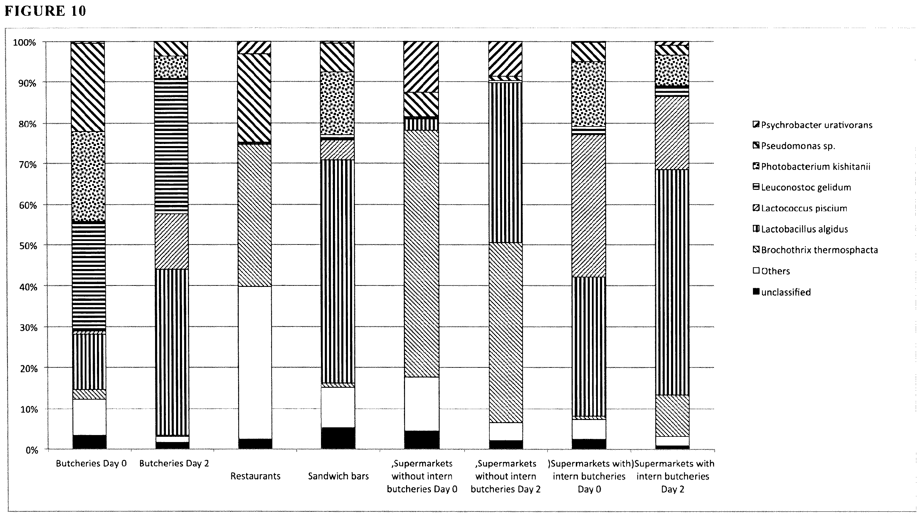

Results of the metagenomic analysis on steak tartare are shown in Figure 10. Each lane represents 100% of the bacterial composition of one sample. Each colour corresponds to a certain amount of sequences assign to a certain taxa.

A total of 32 dominant bacterial species were identified in the steak tartare samples by the metagenomic analysis. *Brochothrix thermosphacta, Lactobacillus algidus, Lactococcus piscium, Leuconostoc gelidum, Photobacterium kishitanii, Pseudomonas sp.* and *Psychrobacter urativorans* are the most predominant bacterial species in the samples and represent 89% of the detected bacterial species.

*Brochothrix thermosphacta and Lactobacillus algidus,* represent 50% of all the detected species. *Brochotrix thermosphacta* is detected mostly in samples from restaurants (2,8-93,9 %; n=4/6) and supermarkets without intern butcheries (0,3-83,2 %; n=8/8). *Lactobacillus algidus* is present in each category but in low proportion for restaurants (2,3-5,5 %, n=3/6). *Pseudomonas sp, Brochotrix thermosphacta* or *Leuconostoc gelidum* are present in high proportion in some samples at Day 0. These bacteria are well known to spoil meat product and samples that contain these bacteria in high proportion are more susceptible to spoil rapidly. Some samples (data not shown) contain a high level of *Streptococcus* or *Enterobacter* resulting probably of cross contamination during the meat process.

A food with a concentration above 6 log CFU/g is generally considered as spoiled. However, the results indicate that some samples have a concentration around this theoretical limit and contains a high proportion of bacteria which are not considered as spoilage bacteria *like Lactobacillus algidus.*

The metagenomic analysis is a powerful tool to identify and to measure the relative proportions of dominant bacterial species in the steak tartare as any other food or non food samples.

Some bacterial species could be indicators of the meat quality. Identification of some bacterial species over time gives information on the evolution of the spoilage process.

Therefore, metagenomic analysis could be an additional tool to control and manage the meat quality as well as the risk of spoilage.

### Semi-quantitative metagenomic analysis

The main drawbacks of the metagenomic approach is the relativity of the proportions of the bacterial populations. The following example shows that linking these proportions to a validated estimated bacterial count is an actual tool to accurately qualify the quality of a food sample.

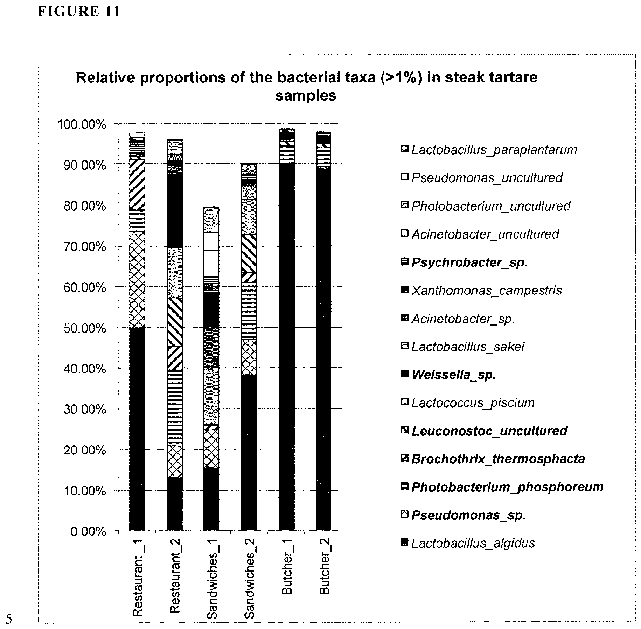

The figure 11 shows the relative proportions of bacteria found in several samples of steak tartare preparation obtained from 2 restaurants, 2 from sandwich vendors and 2 from butcheries. The metagenomic sequencing and analysis followed the same protocol as described above.

The samples are clearly populated with various populations with a leading domination of Lactic Acid Bacteria (LAB). The first taxon is *Lactobacillus algidus,* which is a common bacterium frequently found in cold stored beef meat. This bacterium is homofermentar and not known for any spoilage activity. Another LAB is *Lactococcus piscium* which again is a non-spoiling bacteria.

Other populations observed however are known to possess spoiling activity when their population is high enough. This is the case for *Leuconostoc,* sp., *Weissella* sp., *Photobacterium phosphoreum* and *Brochotrhix thermosphacta.*

We would like to focus on 2 samples: Restaurant-2 and sandwich-2. These samples possess the highest levels of contamination by *Leuconostoc, Weissella* and *Photobacterium* in terms of relative proportions.

Based on these observations, one could argue that both samples are not of good quality because of the presence of spoilage bacteria (*Leuconostoc* giving a cheesy and slimy flavor on the meat). But, in fact these relative proportions don't reflect the true level of bacterial contamination.

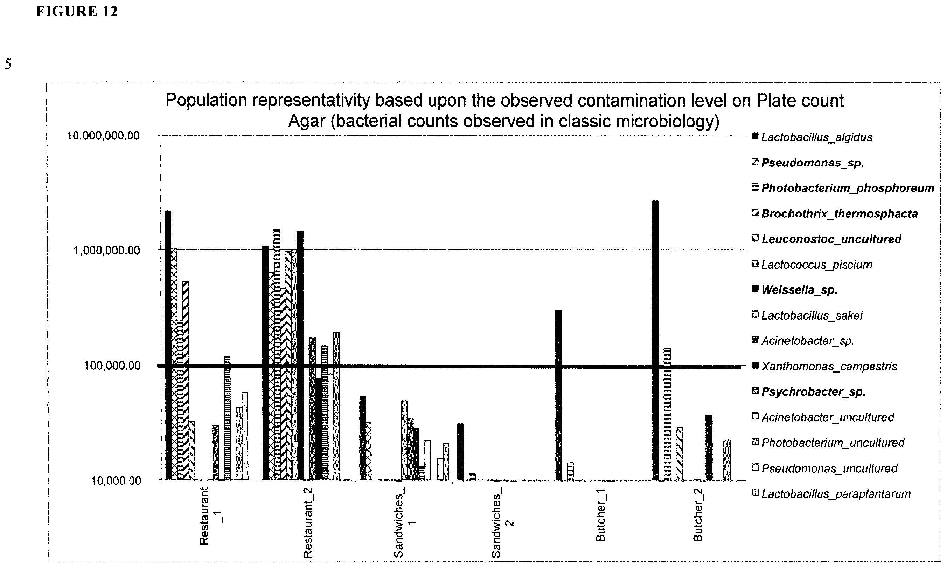

In order to visualize the results from the point of view of a food microbiologist, we convert the OTU populations into estimated log CFUs, taking the following as an arbitrary statement: that the proportion of an OTU to the entire OTU population was related to the same proportion of log CFUs to the log of the total plate count (Figure 12). As we chose matrices actively invaded by bacteria, these were considered as active and thus correctly represented by the colonies grown on plates. This is not a canonical choice but it allows the selection of OTU populations with a size of over 100,000 cells; a threshold above which is said to lead to spoilage for most altering bacteria in foodstuff at the shelf life limit.

In the sample restaurant-2, several spoilage bacteria are present with a population level above 1,000,000 cells per gram. Such level clearly has a noticeable sensory effect.
On the contrary, the sample Sandwich-2 is in fact lowly contaminated. As the total flora count of mesophilic bacteria is below 10,000 cells per gram, it doesn't appear on the graph. Thus the spoilage population, in the event they are present, will not have a sensory effect at the time of sampling.

This combination allows us to clearly see the true impact of the bacteria present.

### Discrimination between dead or alive bacteria

Metagenomics is an extremely valuable tool for probiotics quality control. The methodology of the invention enables the validation of the determined bacterial and/or yeast content of the product. As described above, by adding a PMA treatment to the sample prior to the DNA extraction, the viability of the determined micro-organisms might be assessed.

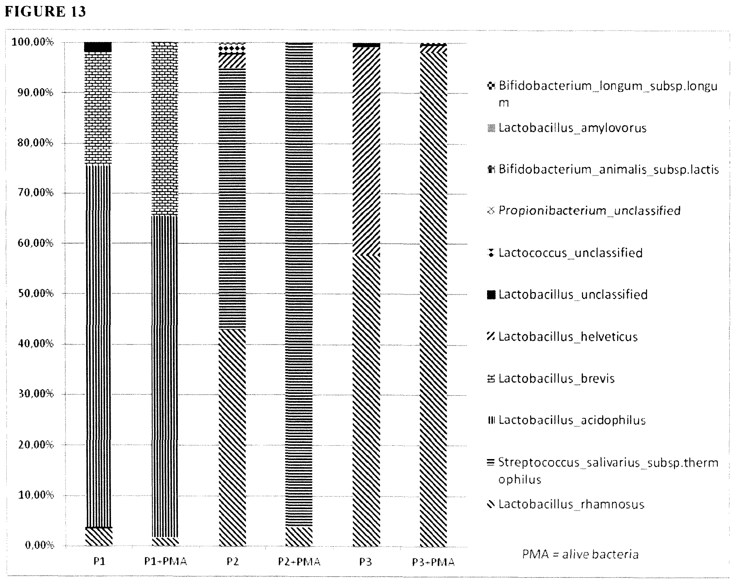

As seen in figure 13, the proportions of some bacterial groups are modified by the PMA treatment. By comparison between a sample PMA treated (Px^{PMA}) or not (Px), one can see that the total amount of certain species is decreasing after a PMA treatment meaning that the analysis conclude positively about the presence of such a bacterial group in the probiotic but underline the fact that the viability of this group is not well conserved. Indeed, the decrease of proportion indicate that part of those bacteria are actually already dead which is not interesting as the effect of a probiotic intake will be due to the gainful activity of the viable bacteria.

### Elimination of plastid and mitochondrial DNA co-amplification.

Since the chloroplast 16S and mitochondrial 18S rRNA genes share high sequence similarity with bacterial 16S rRNA sequences, contamination with plant DNA poses a serious challenge for the application of PCR-based methods to profile and quantify bacterial populations in plant environments. Microbiome profiling of a food product mixed with herbs, spices or any vegetable is really challenging without specific treatment avoiding the amplification of chloroplastic or/of mitochondrial DNA. Without this kind of treatment, the proportion of those "contaminating" DNA could indeed represent in some cases until 99 percent of the reads obtained by the high-throughput sequencing. Considering the cost of an analysis, sequencing 9900 sequences out of 10000 coming out of spices when trying to analyze the bacterial diversity of the product is definitely not what is expected.

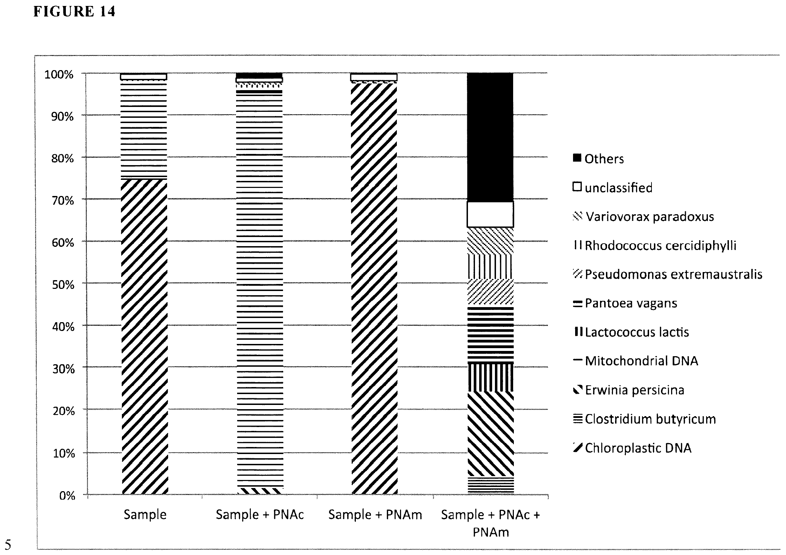

We analyze such a product at the date of production normally meaning with a low amount of bacterial flora. Without any treatment, the majority of the DNA sequences were coming from chloroplastic DNA. Based on the bacterial and chloroplastic DNA alignment described earlier, 2 PNA clamping probes were design to block the amplification of any chloroplastic DNA during the 16S specific PCR. Using those probes, no DNA was amplified from chloroplast but the majority of the produced sequences were from mitochondrial DNA. On the same bases, we then design a PNA clamping probe directed against the amplification of any mitochondrial DNA. By combining the 3 PNA clamping probes, we could finally observe the bacterial diversity of the product (figure 14).

Without those probes, the bacterial diversity of many food or agricultural samples are just impossible to explore.

### Elimination of specific over-represented taxa.

In fermented food, the use of a starter to induce the fermentation has often as a result that those microorganisms that are part of the recipe characterize a major part of the final ecosystem of the product.

In case of quality problem leading to a non-conform product, it is really important to be able to identify the microbial diversity often hidden by the major starter microorganisms. As for vegetable products, the invention is using a PNA clamping probe to block the amplification of those "contaminant" flora to be able to see the diversity behind.

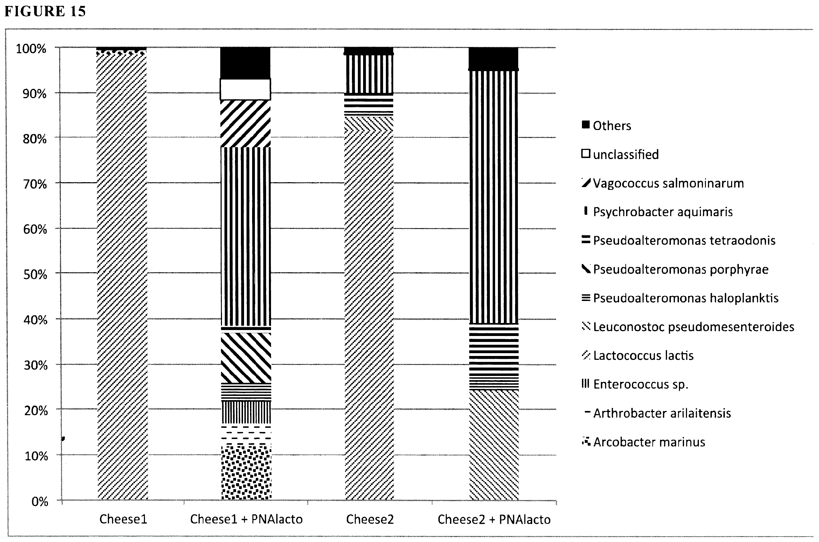

Figure 15 represents the results of a metagenomic analysis of two different cheese cords with and without PNA probe designed against the amplification the lactococcus taxa representing the major proportion of their microbial flora.

*Lactococcus* lactis is introduced thought the starter to initiate the cheese fermentation. In both cheeses, the bacterial profile is dominated by the *lactococcus* genera preventing the underlying diversity to be observed. As can be clearly seen in figure 15, the use of the PNA clamp designed to block the amplification of any *Lactococcus* species has a huge effect on the observed bacterial profile. When PNALactococcus is used in the metagenomic protocols, the *Lactococcus* genus clearly disappeared in favour of the underlying diversity.

### Conclusion

Metagenomic analysis offers a new tool for identifying microorganisms present in perishable food and their evolution depending of environmental conditions. The information available through metagenomics analysis provides a clear picture of the microbial community. Microbiological ecology studies have shown that the bacterial world of foods are much more diverse than the cultivable group of bacteria studied by culture media. For the metagenomic analysis, 3 to 5 thousand sequences appeared largely sufficient to identify and to characterize the spoiling microflora, though that might change depending on the objective and the budget of a particular study. Many food manufacturers, government agencies, retailers, distribution quality laboratories and researchers use general culture media without precisely knowing the bacterial communities inside the food. Compared to culture based methods on selective media and previous independent culture techniques, metagenomic analysis combined with the enumeration of psychrotrophic flora gives more valuable information, and its use should be considered as a technique for quality control, for accurately determining shelf life or for developing new food products.

The invention relates to a global analytical tool that is especially useful in the food industry to identify and quantify the spoilage microbiota responsible of organoleptic alterations of the food.

The term metagenomics could be defined as "the application of modern genomics techniques to the study of communities of microbial organisms directly in their natural environments, bypassing the need for isolation and lab cultivation of individual species" Conventional sequencing begins with a culture of identical cells as a source of DNA. However, early studies revealed that there are probably large groups of microorganisms in many environments that cannot be cultured and thus cannot be sequenced.

For environmental studies, the interest in metagenomics is to analyse the complete bacterial diversity to determine the type and relative abundance of any populations present in heterogeneous samples, such as soil, marine, or gut microbiome.

The metagenomic analysis of the present invention may be used as a tool for food safety and/or for food quality control. Described is a package of different analytical options allowing analysis to only focus on the viable spoilage flora specific to a particular kind of food sample. The present methods can be used whether the sample is: raw or cooked; with or without addition of spices or any kind of vegetables in the recipe; with or without the use of a bacterial starter to boost the fermentation process; and/or with or without added protective flora for extension of the product shelf life.

Unlike environmental samples, food samples are potentially subjected to different treatments like: Thermic treatment such as cooking, sterilisation, pasteurisation.

Those thermic treatments will kill most of the bacterial flora present in the food but the DNA will still be present and taken into account in the metagenomic description of the potential alteration cause of the cooked product and could induce a false interpretation of the spoilage origin.

Those cooked products should be more or less free of spoilage bacteria but could be subject to a post thermic treatment contamination.

### Fermentation

A starter culture can be defined as a microbial preparation of large numbers of cells of at least one microorganism to be added to a raw material to produce a fermented food by accelerating and steering its fermentation process.

The use of starter culture shortens the fermentation process and reduces the risk of fermentation failure.

As a consequence, if the producer requires a metagenomic analysis of fermented food (cheese, kefir, yogurt, salami, wine, beer, sourdough,...) to check for contamination or spoilage flora, the present methods are able to detect sequences of the flora of interest through the use of PNA specifically added in the PCR reaction to block the amplification of unwanted DNA originating from the starter flora.

### Addition of flora for a biological preservation of the food.

The addition of a large amount of protecting flora, that is bacteria not associated to any spoilage process in a specific kind of food sample, will contribute to decrease the development of the unintended spoilage flora.

Our invention will in this case also give the opportunity to cancel the amplification of the DNA originating from the protective flora through the use of specifically targeted PNA.

### Addition of spices or vegetables in the recipe

In prepared food with added spices or vegetables in the recipe, the sequences of spoilage bacterial 16S rDNA are often hidden under an overabundance of sequences produced by co-amplification of the chloroplastidial and mitochondrial 16S rDNA.

The presence of mitochondrial and plastid DNA in those kinds of samples has limited the full realization of the potential offered by the last generation of metagenomics approaches dedicated to the feed and food industry.

The present invention will in this case also give the opportunity to cancel the co-amplification of the DNA originating from spices and/or vegetables through the use of specifically targeted PNA.

The present invention provides for the quantification of the identified spoilage bacteria by the use of our metagenomic analysis.

The food product contains some bacteria that are involved in spoilage, the so-called specific spoilage organisms (SSO), and others which grow without causing unpleasant changes. Depending of the food matrix, it is usually assumed that the spoilage process begins with a concentration of total flora higher than 5-6 log cfu/g. The SSO may be present at a low concentration and, in that case, are not able to spoiled the product.
The result of a metagenomic study is the identification of the different observed bacteria with their relative proportions and is therefore only semi-quantitative.

To correctly interpret the data from the ultra-sequencing it is crucial to develop a parallel method to estimate the concentration of the different bacterial species present in the food sample.

If coupled with the results of enumeration of total bacteria in conventional culture medium, the relative proportions obtained by metagenomic analysis might give an estimation of the real proportion of the different observed species in terms on cfu per gram of samples.

However, one drawback of conventional microbiological methods lies in their failure to detect some of the existing microflora present in a food matrix resulting in an underestimation of the contamination levels of microorganisms.

To get an accurate and reliable counting of the metagenomic profile, the present invention relates to the use of newly developed quantitative real-time PCR (qPCR) of the various taxa predominantly present in food samples.

The present invention relates therefore to the estimation of the real amount of the different taxa identified in the metagenomic profile based of the real quantification of one of them by qPCR giving an estimate of bacterial concentration by far more accurate and reliable.

### PNA

A food technological flora is a flora that brings more value to the product (texture, flavoring, acidification, nutritional properties, stabilization and conservation). This flora is often added during the manufacturing process of the food mastered in proportions so as to achieve the desired result in the finished product. In the food industry adding useful flora is a very common process.

This use might be a problem when the spoilage origin is to be analyzed through the use of a food metagenomic analysis. Indeed, if a food has been altered, the purpose of the metagenomic analysis will be to provide a description of the flora that might be responsible of this alteration. In the case of any use of technological flora during the process, the DNA of it might be present at more than 95 % preventing the spoilage flora' DNA from being observed.

In a food metagenomic analysis, the use of specific PNA acting against the amplification of any "contaminant" DNA belonging to any specific technologic flora is therefore importance.

### PNA Bacillus

In spite of spore forming strains, the genus bacillus is widely used as starter in food fermentation. In Africa, *Bacillus subtilus, Bacillus pumilus* and *Bacillus Licheniformis* are involved in bean fermentation. In Japan, Natto, a unique traditional health food, is made using a fermentation process by adding beneficial bacteria, *Bacillus natto* to soybeans.

The present invention relates to the development of a PNA blocking the amplification of any bacillus species used as or within a technologic flora.
The accuracy of the PNA design has been checked with the PNA designer of "Life Technologies". (http://www6.appliedbiosystems.com/support/pnadesigner.cfm)

**Table 1**

| **Calculation results** | |
|---|---|
| Sequence | Lys-AGA CTG GGA TAA CTC CG |
| N Terminus Reporter | N: None |
| C Terminus Reporter | C: None |
| Tm | [1 µM] = 80 °C |
| | [1 nM] = 69 °C |
| | [1 pM] = 59 °C |
| Molecular Weight | 4782,27 |
| Length | Residues: 18 |
| | Bases: 17 |
| % Purine | 58,80% |
| Purine Stretch | 4 |
| Complementarity | Moderate |
| | AGACTG**GGA**TAAC**TCC**G |
| Design Recommendations | Design Suitable |

### PNA Streptococcus thermophilus + PNA Lactobacillus delbrueckii

In the milk processing industry, starter cultures and ferments are widely used particularly in the manufacture of yoghurt to provide a particular organoleptic aspect (acidification, flavour, texture etc...)

According to the Codex Alimentarius (A-lla of 1975), yogurt is a coagulated milk product resulting from the fermentation of fresh milk or pasteurized milk, with or without other added ingredients, thanks to the action of *Lactobacillus delbueckii* subsp. *bulgaricus* and *Streptococcus thermophilus.* In the finished product to have the "yogurt" designation, those 2 bacteria must be viable and abundant in quantity.

However, microbial diversity of such products may be masked by the presence of a large proportion of this technological flora. Thereby investigation of the diversity through the use of molecular techniques may be hindered by the very high concentration of these two technological flora. It is therefore necessary to find or develop an adequate technique to circumvent this problem. The easiest way to address this problem is the exclusion at the analytical level of technological flora so that other flora, present at lower levels can be detected and to provide a more complete mapping of the microbial alteration of the product. The negative aspect of this approach is that the majority (sometimes > 99 %) of the generated sequences will come from those 2 bacteria and will therefore be irrelevant to explore the alteration of the product.

Another way is to block the specific DNA amplification of those 2 bacteria by adding 2 PNA molecules in the PCR reaction.

### Design PNA Streptococcus thermophiles

The accuracy of the PNA design has been checked with the PNA designer of "Life Technologies", (http://www6.appliedbiosystems.com/support/pnadesigner.cfm).

**Table 2**

| **Calculation Results** | |
|---|---|
| Sequence | Cys-ACC CTG CCT TGT AGC GGG |
| N Terminus Reporter | N: None |
| C Terminus Reporter | C: None |
| Tm | [1 µM] = 84 °C |
| | [1 nM] = 74 °C |
| | [1 pM] = 64 °C |
| Molecular Weight | 5015,5 |
| Length | Residues: 19 |
| | Bases: 18 |
| % Purine | 50,00% |
| Purine Stretch | 3 |
| Complementarity | Low |
| | AACCTGCCTTGTAGCGGG |
| Design Recommendations | Design Suitable |

### Design PNA Lactobacillus delbrueckii

The accuracy of the PNA design has been checked with the PNA designer of "Life Technologies". (http://www6.appliedbiosystems.com/support/pnadesigner.cfm).

**Table 3**

| **Calculation Results** | |
|---|---|
| Sequence | Lys-TAA CAA CAT GAA TCG CAT |
| N Terminus Reporter | N: None |
| C Terminus Reporter | C: None |
| Tm | [1 µM] = 74 °C |
| | [1 nM] = 65 °C |
| | [1 pM] = 56 °C |
| Molecular Weight | 5000,57 |
| Length | Residues: 19 |
| | Bases: 18 |
| % Purine | 55,60% |
| Purine Stretch | 3 |
| Complementarity | Moderate |
| | TAACAA**CATG**AATCGCAT |
| Design Recommendations | Design Suitable |

### The use of quantitative PCR to deduce total flora and metagenomic data.

In prior art methods the relative proportions obtained by metagenomic analysis have been coupled with total flora enumeration from classic microbiology. This allows an estimation of the quantity of the major microorganisms present in a food sample.

However, these methods cause some biased results because numerous microorganisms cannot be cultivated. Enumeration data are thus widely misestimated.

To get a precise and reliable enumeration, it's important to work out an alternative technique to classical microbiology by quantitative PCR to detect and quantify some major taxons from foodstuffs.

The list of taxons to identify by qPCR has been obtained from previous metagenomic analyses of data on various food samples. These taxons were chosen based on their appearance frequency and their impact on the organoleptic qualities of the food.

The 7 taxons mainly present in classic perishable foodstuffs selected are:
- *Lactobacillus* sp.
- *Lactococcus* sp.
- *Leuconostoc* sp.
- *Psychrobacter* sp.
- *Pseudomonas* sp.
- *Brochrohtrix* sp.
- *Photobacterium* sp.

All the target genes used for the 7 qPCR tests belong to the conserved "housekeeping" genes, present in single copy in the bacterial genome, an important feature for the quantification method, allowing the design of "genus" common primers and hydrolysis probes allowing the relative proportion estimate of a majority of species included in a same bacterial genus (table 1).

**Table 4 : Listing of primers and probes designed for the qPCR tests allowing the relative proportion estimate of 7 taxons mainly present in classic perishable foodstuffs :**

| target bacterial genus | target gene | primers | sequence |
|---|---|---|---|
| Lactobacillus sp | tuf | Lactobacillus-Tuf-F2 | 5'-GCYCACGTWGAATAYGAAAC-3' |
| | | Lactobacillus-Tuf-R2 | |
| | | Lactobacillus-Tuf-FAM1 | |
| Lactococcus sp | rpoA | Lactococcus-RecA-F2 | 5'-GCCGAAATYGATGGYGAAAT-3' |
| | | Lactococcus-RecA-R2 | |
| | | Lactococcus-RecA-FAM4 | |
| Leuconostoc sp | fus | Leuconostoc-Fus-F 1 | |
| | | Leuconostoc-Fus-R1 | |
| | | Leuconostoc-Fus-FAM1 | |
| Photobacterium sp | rpoA | photobactreium-rpoA-F 1 | 5'-AATGCCAGGTTGTGCTG-3' |
| | | photobactreium-rpoA-R 1 | |
| | | photobactreium-rpoA-FAM1 | |
| Pseudomonas sp | rpoA | Pseudo-rpo-F2 | 5'-GGCGTKATCGAYTCSGT-3' |
| | | Pseudo-rpo-R2 | 5'-CAGMGGRCGCTGGTTGATG-3' |
| | | Pseudo-rpo-FAM2 | 5'-TCGYGTTGCYGAYGACGA-3' |
| Carnobacterium sp | rpoA | Carnobacterium-rpoA-F1 | 5'-ATTGGYGTATTACCAGTCGA-3' |
| | | Carnobacterium-rpoA-Rl | 5' -AACCA TCTGCCCA T ACA TC-3' |
| | | Carnobacterium-rpoA-FAM1 | |
| Brochothrix sp | tuf | Brochotrix-tuf-Fl | 5'-CAGTACCTTCTGGTAACGTG-3' |
| | | Brochotrix-tuf-Rl | |
| | | Brochotrix-tuf-FAM | |

### Conventional PCR protocol

Real-time PCR was performed using the LightCycler 480 system (Roche, Basel, Switzerland). The PCR reaction mixtures were placed in a 12 µl final volume containing 6 µl of LC480 probe master mix (Roche, Basel, Switzerland), 2 µl of template DNA (at 5ng/µl), 0,25 µl of primer pair (10µM each), 0,125 µl of Taqman probe (10µM). The reaction conditions included the initiation step for 10 min at 95°C, followed by 40 cycles of 15s at 95°C and 1 min at 60°C. The real-time system is supplied with the Lightcycler 480 Software version 1.5 using unique Roche algorithms for highly accurate and robust automated data analysis. Serial dilutions (10⁶ to 1 copy numbers) of standard DNA are used for determining bacterial number. The mean of Cycle Threshold (CT) on the three repetition was used in order to estimate the load of targets bacterial populations present in the sample.

### Specificity and sensitivity test.

The specificity of each genus qPCR were tested on close bacterial species which may be present in foodstuffs to verify if each test does not interact with other bacterial species phylogenetically close (table 5).

In sensitivity test of the specific primers and probes, PCR amplifications were examined between specific primers and 10-fold serial dilutions of DNAs isolated from pure culture of 3 target species belongs to the target bacterial genus ranging from 10⁶ to 10 copy numbers. The standard curves for all the 6 detection systems were constructed by using the Cp value obtained from the corresponding DNA concentration.

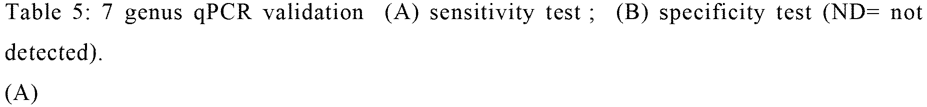

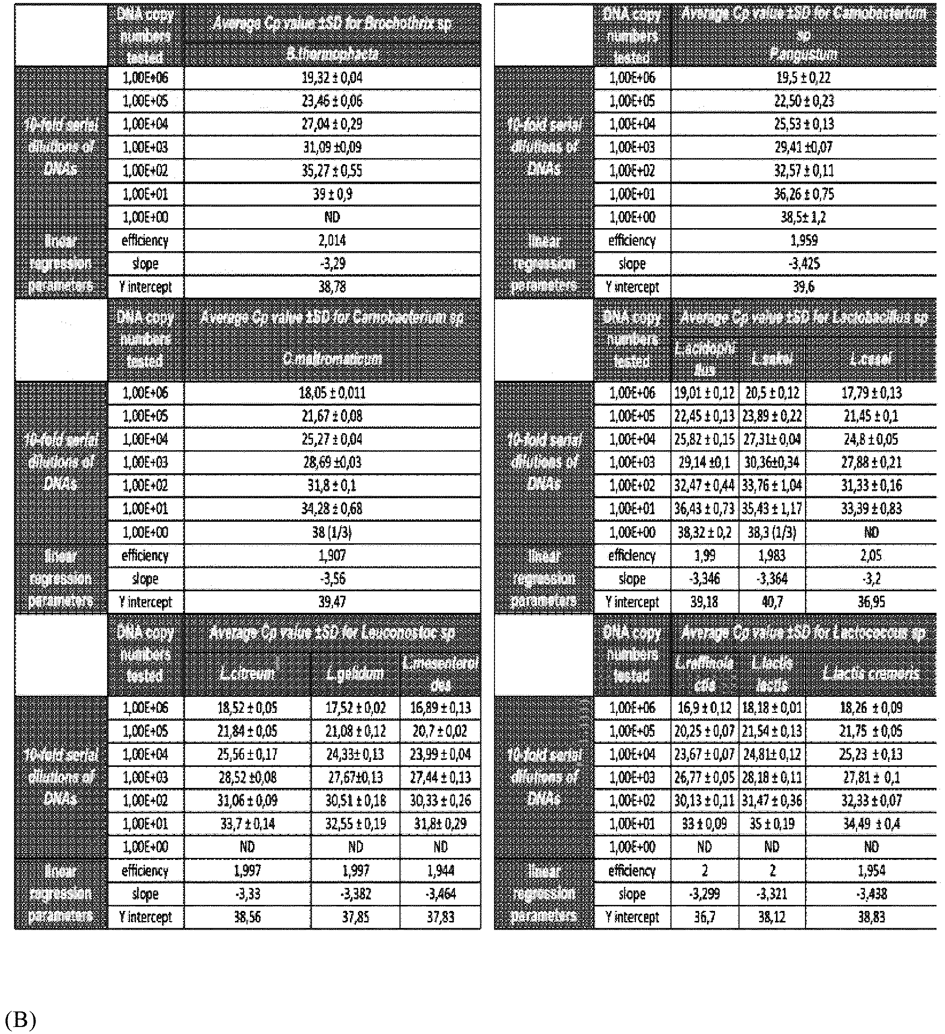

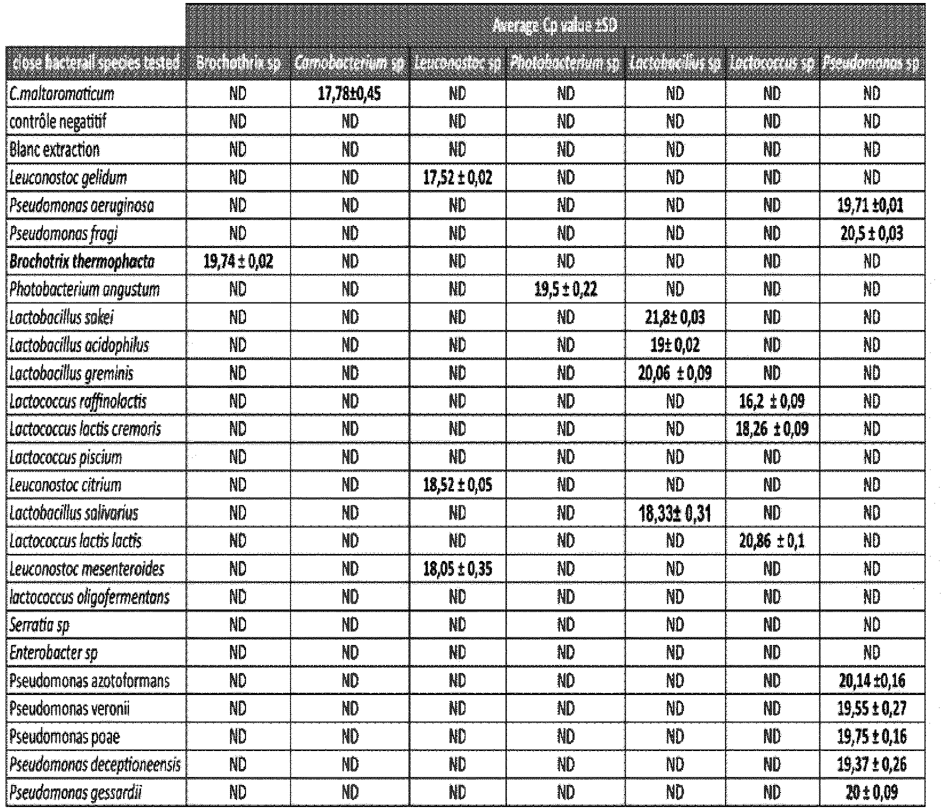

### Correlation between classic microbiology, targeted metagenomic and quantitative data : example on on real foodstuff samples.

6 naturally contaminated matrices from different origins (white pudding, pancake, hamburger, ham, pasta, cheese) were analyzed by microbiology, metagenomic analysis and quantitative PCR to estimate proportion of some major taxons (*Carnobacterium* sp, *Leuconostoc* sp, *Photobacterium* sp, *Brochotrix* sp, *Pseudomonas* sp, *Lactococcus* sp et *Lactobacillus* sp). Among the 7 taxons tests in qPCR, 3 are detected in all the 6 food samples (*Leuconostoc* sp, *Lactococcus* sp et *Lactobacillus* sp). Thus, the total bacterial flora could be deduced using these qPCR data. The total flora estimated by classic microbiology is always widely mis-estimated compared to total flora deduced by qPCR (fig 19). The qPCR method allow to estimate more accurately total bacterial flora (including cultivated or not cultivated bacteria).

These deduction of total flora by qPCR method will allow to quantify more precisely all the taxons identified by metagenomic analysis (fig 20). The figure 20 (A) shows the bacterial profile obtained by metagenomic analysis for the 6 food samples. These analyses don't allow estimation of the concentration of each taxon denominated in colony-forming units per gram of matrix (cfu/g). The total flora estimation by quantitative PCR combining with the percentage of reads from genomic analysis allow to obtain an estimation of each taxon concentration in colony-forming unit per gram of matrix (fig 20(B).

The invention is not limited by the examples or figures provided herein. The skilled person is able to conceive of alternatives for the various sequences, tools and methods mentioned in this specification. The invention is limited only by the scope of the claims.

## Claims

1. A method of metagenomic analysis of fermented food to detect and quantitate bacteria present in said sample, the analysis comprising amplification of the VI-V3 region of bacterial 16S rRNA in said sample, wherein the V1-V3 region is analysed with the primer pair of SEQ ID NO.1 and SEQ ID NO. 2, wherein the method further comprises the use of Peptide Nucleic Acid (PNA) clamping to reduce amplification of at least one of *S. thermophilus* and *L. delbrueckii, Lactococcus* or *Bacillus* which may be present in the sample.

2. A method of claim 1 wherein propidium monoazide (PMA) is added to the sample prior to DNA extraction in order to reduce the subsequent amplification of DNA from non-viable bacteria present in the sample.

3. A method of claim 1 or claim 2 wherein said analysis can detect and distinguish bacteria of the families: *Acetobacteraceae, Aeromonadaceae, Bacteroidaceae, Burkholderiaceae, Carnobacteriaceae, Clostridiaceae, Comamonadaceae, Enterobacteriaceae, Enterococcaceae, Flavobacteriaceae, Lachnospiraceae, Lactobaillaceae, Leuconostocaceae, Listeriaceae, Moraxellaceae, Neisseriaceae, Pseudoalteromonadaceae, Pseudomonadaceae, Shewanellaceae, Streptococcaceae, Vibrionaceae* and *Xanthomonadaecea;* and/or
wherein said analysis can detect and distinguish bacteria of the genuses: *Acetobacteraceae Acetobacter, Acetobacteraceae Gluconacetobacter, Aeromonadaceae Aeromonas, Bacteroidaceae Bacteroides,*
*Burkholderiaceae Burkholderia, Carnobacteriaceae Carnobacterium, Clostridiaceae Clostridium, Comamonadaceae Comamonas, Comamonadaceae Delftia, Enterobacteriaceae Escherichia, Enterobacteriaceae Pantoea, Enterobacteriaceae Serratia, Enterococcaceae Enterococcus, Chryseobacterium, Flavobacteriaceae Flavobacterium, Flavobacteriaceae Myroides, Lachnospiraceae Butyrivibrio, Lactobaillaceae Lactobacillus, Lactobacillaceae Pediococcus, Leuconostocaceae Leuconostoc, Leuconostocaceae Weissella, Listeriaceae Listeria, Moraxellaceae Acinetobacter, Moraxellaceae Psychrobacter, Neisseriaceae Alysiella, Pseudoalteromonadaceae Pesudoalteromonas, Pseudomonadaceae Pseudomonas, Shewanellaceae Shewanella, Streptococcaceae Lactococcus, Streptococcaceae Streptococcus, Vibrionaceae Aliivibrio, Vibrionaceae Photobacterium, Vibrionaceae Vibrio* and *Xanthomonadaecea Xanthomonas.*

4. A method of any preceding claim wherein the percentage of bacteria represented by at least one operational taxonomic unit (OTU) is determined, and
optionally further comprising determining the colony forming units count (CFU) of the sample and normalising the percentage of organisms represented by said OTU to a proportion of the total viable CFU count of the sample.

5. A method of any preceding claim wherein the said metagenomic analysis is followed by taxa specific qPCR.

6. A method of any preceding claim wherein said primers are modified by the addition of an adaptor, key sequence, tag or universal sequence, optionally having the sequences of SEQ ID NO.s 82 and 83 respectively.

7. A kit for metagenomic analysis of a sample comprising one or both primers comprising the sequence of SEQ ID NO. 1 and/or SEQ ID NO. 2, preferably wherein the primers consist of the sequences of SEQ ID NO. 1 and/or SEQ ID NO. 2 and further comprising a PNA oligonucleotide comprising the sequence of of SEQ ID NO. 80, preferably wherein the PNA oligonucleotide consists of the sequence of SEQ ID NO. 80.

8. A kit of claim 7 wherein the primer(s) are modified by the addition of an adaptor, key sequence, tag or universal sequence, optionally having the sequences of SEQ ID NO.s 82 and 83 respectively.

9. A kit of any of claims 7 to 8 further comprising propidium monoazide (PMA); and/or further comprising a reagent for metagenomic analysis of a sample.

## Patentansprüche

1. Verfahren zur metagenomischen Analyse fermentierter Lebensmittel zum Nachweisen und Quantifizieren von in der Probe vorliegenden Bakterien, wobei die Analyse Amplifikation der V1-V3-Region von bakterieller 16S-rRNA in der Probe umfasst, wobei die V1-V3-Region mit dem Primerpaar unter SEQ ID NO. 1 und SEQ ID NO. 2 analysiert wird, wobei das Verfahren ferner die Verwendung von PNA(Peptide Nucleic Acid)-Clamping zur Reduzierung der Amplifikation von wenigstens einem von *S. thermophilus* und *L. delbrueckii, Lactococcus* oder *Bacillus,* die möglicherweise in der Probe vorhanden sind, umfasst.

2. Verfahren nach Anspruch 1, wobei die Probe vor der DNA-Extraktion mit Propidiummonoazid (PMA) versetzt wird, um die anschließende Amplifikation von DNA aus in der Probe vorhandenen nicht lebensfähigen Bakterien zu reduzieren.

3. Verfahren nach Anspruch 1 oder Anspruch 2, wobei sich mit der Analyse Bakterien der folgenden Familien nachweisen und unterscheiden lassen: *Acetobacteraceae, Aeromonadaceae, Bacteroidaceae, Burkholderiaceae, Carnobacteriaceae, Clostridiaceae, Comamonadaceae, Enterobacteriaceae, Enterococcaceae, Flavobacteriaceae, Lachnospiraceae, Lactobaillaceae, Leuconostocaceae, Listeriaceae, Moraxellaceae, Neisseriaceae, Pseudoalteromonadaceae, Pseudomonadaceae, Shewanellaceae, Streptococcaceae, Vibrionaceae* und *Xanthomonadaecea;* und/oder
wobei sich mit der Analyse Bakterien der folgenden Gattungen nachweisen und unterscheiden lassen: *Acetobacteraceae Acetobacter, Acetobacteraceae Gluconacetobacter, Aeromonadaceae Aeromonas, Bacteroidaceae Bacteroides, Burkholderiaceae Burkholderia, Carnobacteriaceae Carnobacterium, Clostridiaceae Clostridium, Comamonadaceae Comamonas, Comamonadaceae Delftia, Enterobacteriaceae Escherichia, Enterobacteriaceae Pantoea, Enterobacteriaceae Serratia, Enterococcaceae Enterococcus, Chryseobacterium, Flavobacteriaceae Flavobacterium, Flavobacteriaceae Myroides, Lachnospiraceae Butyrivibrio, Lactobaillaceae Lactobacillus, Lactobacillaceae Pediococcus, Leuconostocaceae Leuconostoc, Leuconostocaceae Weissella, Listeriaceae Listeria, Moraxellaceae Acinetobacter, Moraxellaceae Psychrobacter, Neisseriaceae Alysiella, Pseudoalteromonadaceae Pesudoalteromonas, Pseudomonadaceae Pseudomonas, Shewanellaceae Shewanella, Streptococcaceae Lactococcus, Streptococcaceae Streptococcus, Vibrionaceae Aliivibrio, Vibrionaceae Photobacterium, Vibrionaceae Vibrio* und *Xanthomonadaecea Xanthomonas.*

4. Verfahren nach einem vorhergehenden Anspruch, wobei der durch wenigstens eine OTU (Operational Taxonomic Unit) repräsentierte Prozentsatz Bakterien bestimmt wird und
gegebenenfalls ferner umfassend Bestimmen der CFU(Colony Forming Units)-Zahl der Probe und Normieren des durch die OTU repräsentierten Prozentsatzes Organismen auf einen Anteil der Gesamtzahl lebensfähiger CFU der Probe.

5. Verfahren nach einem vorhergehenden Anspruch, wobei sich an die metagenomische Analyse taxaspezifische qPCR anschließt.

6. Verfahren nach einem vorhergehenden Anspruch, wobei die Primer durch die Addition eines Adaptors, einer Schlüsselsequenz, eines Tag bzw. einer Universalsequenz modifiziert sind, wobei sie gegebenenfalls die Sequenzen unter SEQ ID NO. 82 bzw. 83 aufweisen.

7. Kit zur metagenomischen Analyse einer Probe, umfassend einen oder beide die Sequenz unter SEQ ID NO. 1 und/oder SEQ ID NO. 2 umfassende/n Primer, vorzugsweise wobei die Primer aus den Sequenzen unter SEQ ID NO. 1 und/oder SEQ ID NO. 2 bestehen, und ferner umfassend ein PNA-Oligonukleotid, das die Sequenz unter SEQ ID NO 80 umfasst, vorzugsweise wobei das PNA-Oligonukleotid aus der Sequenz unter SEQ ID NO 80 besteht.

8. Kit nach Anspruch 7, wobei der bzw. die Primer durch die Addition eines Adaptors, einer Schlüsselsequenz, eines Tag bzw. einer Universalsequenz modifiziert ist bzw. sind, wobei er bzw. sie gegebenenfalls die Sequenzen unter SEQ ID NO. 82 bzw. 83 aufweist bzw. aufweisen.

9. Kit nach einem der Ansprüche 7 bis 8, ferner umfassend Propidiummonoazid (PMA); und/oder ferner umfassend ein Reagens zur metagenomischen Analyse einer Probe.

## Revendications

1. Méthode d'analyse métagénomique d'aliments fermentés afin de détecter et de quantifier des bactéries présentes dans ledit échantillon, l'analyse comprenant l'amplification de la région V1-V3 de l' ARNr 16S bactérien dans ledit échantillon, où la région V1-V3 est analysée avec la paire d'amorces de SEQ ID n° 1 et SEQ ID n° 2, la méthode comprenant en outre l'utilisation d'un clamping à base d'Acide Peptidonucléique (APN) afin de réduire l'amplification d'au moins un parmi *S. thermophilus* et *L. delbrueckii, Lactococcus* ou *Bacillus* pouvant être présents dans l'échantillon.

2. Méthode selon la revendication 1, dans laquelle du propidium monoazide(PMA) est ajouté à l'échantillon préalablement à l'extraction d'ADN afin de réduire l'amplification ultérieure d'ADN issu de bactéries non viables présentes dans l'échantillon.

3. Méthode selon la revendication 1 ou la revendication 2, dans laquelle ladite analyse peut détecter et distinguer des bactéries issues des familles : *Acetobacteraceae, Aeromonadaceae, Bacteroidaceae, Burkholderiaceae, Carnobacteriaceae, Clostridiaceae, Comamonadaceae, Enterobacteriaceae, Enterococcaceae, Flavobacteriaceae, Lachnospiraceae, Lactobaillaceae, Leuconostocaceae, Listeriaceae, Moraxellaceae, Neisseriaceae, Pseudoalteromonadaceae, Pseudomonadaceae, Shewanellaceae, Streptococcaceae, Vibrionaceae* et *Xanthomonadaceae* ; et/ou
où ladite analyse peut détecter et distinguer des bactéries issues des genres :
*Acetobacteraceae Acetobacter, Acetobacteraceae Gluconacetobacter, Aeromonadaceae Aeromonas, Bacteroidaceae Bacteroides, Burkholderiaceae Burkholderia, Carnobacteriaceae Carnobacterium, Clostridiaceae Clostridium, Comamonadaceae Comamonas, Comamonadaceae Delftia, Enterobacteriaceae Escherichia, Enterobacteriaceae Pantoea, Enterobacteriaceae Serratia, Enterococcaceae Enterococcus, Chryseobacterium, Flavobacteriaceae Flavobacterium, Flavobacteriaceae Myroides, Lachnospiraceae Butyrivibrio, Lactobacillaceae Lactobacillus, Lactobacillaceae Pediococcus, Leuconostocaceae Leuconostoc, Leuconostocaceae Weissella, Listeriaceae Listeria, Moraxellaceae Acinetobacter, Moraxellaceae Psychrobacter, Neisseriaceae Alysiella, Pseudoalteromonadaceae Pesudoalteromonas, Pseudomonadaceae Pseudomonas, Shewanellaceae Shewanella, Streptococcaceae Lactococcus, Streptococcaceae Streptococcus, Vibrionaceae Aliivibrio, Vibrionaceae Photobacterium, Vibrionaceae Vibrio* et *Xanthomonadaecea Xanthomonas.*

4. Méthode selon l'une quelconque des revendications précédentes, dans laquelle le pourcentage de bactéries représenté par au moins une OTU (Operational Taxonomic Unit) est déterminé, et
éventuellement comprenant en outre la détermination du comptage de CFU (Colony Forming Units) de l'échantillon et la normalisation du pourcentage d'organismes représentés par ladite OTU vis-à-vis d'une proportion du comptage de CFU viable total de l'échantillon.

5. Méthode selon l'une quelconque des revendications précédentes, dans laquelle ladite analyse métagénomique est suivie d'une qPCR spécifique des taxons.

6. Méthode selon l'une quelconque des revendications précédentes, dans laquelle lesdites amorces sont modifiées par l'addition d'un adaptateur, d'une séquence clef, d'une étiquette ou d'une séquence universelle, éventuellement répondant aux séquences de SEQ ID n° 82 et 83, respectivement.

7. Kit destiné à l'analyse métagénomique d'un échantillon comprenant une, ou les deux, amorces comprenant les séquences de SEQ ID n° 1 et/ou SEQ ID n° 2, préférablement où les amorces sont constituées des séquences de SEQ ID n° 1 et/ou SEQ ID n° 2, et comprenant en outre un oligonucléotide d'APN comprenant la séquence de SEQ ID n° 80, préférablement où l'oligonucléotide d'APN est constitué de la séquence de SEQ ID n° 80.

8. Kit selon la revendication 7, dans lequel la ou les amorces sont modifiées par l'addition d'un adaptateur, d'une séquence clef, d'une étiquette ou d'une séquence universelle, éventuellement répondant aux séquences de SEQ ID n° 82 et 83, respectivement.

9. Kit selon l'une quelconque des revendications 7 à 8, comprenant en outre du propidium monoazide (PMA) ; et/ou comprenant en outre un réactif destiné à l'analyse métagénomique d'un échantillon.
